# EUROPÄISCHE PATENTSCHRIFT

(11) **EP 2 550 353 B1**
(45) Veröffentlichungstag und Bekanntmachung des Hinweises auf die Patenterteilung: **24.05.2017**
(21) Anmeldenummer: 11709885.5
(22) Anmeldetag: 21.03.2011
(51) Int. Cl.: C12N 5/00

(54) **THERMORESPONSIVES SUBSTRAT MIT MIKROGELEN, VERFAHREN ZU DESSEN HERSTELLUNG UND KULTIVIERUNGSVERFAHREN FÜR BIOLOGISCHE ZELLEN**
THERMOREACTIVE SUBSTRATE WITH MICROGELS, METHOD FOR ITS PREPARATION, AND CULTURE METHOD FOR BIOLOGICAL CELLS
SUBSTRAT THERMORÉACTIF COMPORTANT DES MICROGELS, PROCÉDÉ DE FABRICATION DE CE SUBSTRAT ET PROCÉDÉ DE CULTURE DE CELLULES BIOLOGIQUES

(30) Priorität: 22.03.2010 DE 102010012252
(43) Veröffentlichungstag der Anmeldung: 30.01.2013
(73) Patentinhaber: Fraunhofer-Gesellschaft zur Förderung der angewandten Forschung e.V., 80686 München (DE)
(72) Erfinder: DUSCHL, Claus, 10115 Berlin (DE); LANKENAU, Andreas, 6343 Rotkreuz (CH); LUTZ, Jean-François, 77694 Kehl (DE); LASCHEWSKY, André, 14469 Potsdam (DE); WISCHERHOFF, Erik, 14469 Potsdam (DE); SCHMIDT, Stephan, 14471 Postdam (DE); HELLWEG, Thomas, 33615 Bielefeld (DE)
(74) Vertreter: Katzameyer, Michael
(86) Internationale Anmeldenummer: PCT/EP2011/001394
(87) Internationale Veröffentlichungsnummer: WO 2011/116922

(56) Entgegenhaltungen:
- SCHMIDT S ET AL: "Adhesion and Mechanical Properties of PNIPAM Microgel Films and Their Potential Use as Switchable Cell Culture Substrates", ADVANCED FUNCTIONAL MATERIALS, WILEY - V C H VERLAG GMBH & CO. KGAA, DE, Bd. 20, Nr. 19, 8. Oktober 2010 (2010-10-08), Seiten 3235-3243, XP002604262, ISSN: 1616-301X, DOI: 10.1002/ADFM.201000730 [gefunden am 2010-08-04]
- SCHMIDT ET AL: "Thermoresponsive surfaces by spin-coating of PNIPAM-co-PAA microgels: A combined AFM and ellipsometry study", POLYMER, ELSEVIER SCIENCE PUBLISHERS B.V, GB, Bd. 49, Nr. 3, 23. Dezember 2007 (2007-12-23), Seiten 749-756, XP022436035, ISSN: 0032-3861, DOI: 10.1016/J.POLYMER.2007.12.025
- STEPHAN SCHMIDT ET AL: "Packing Density Control in P(NIPAM-co-AAc) Microgel Monolayers: Effect of Surface Charge, pH, and Preparation Technique", LANGMUIR, Bd. 24, Nr. 21, 4. November 2008 (2008-11-04), Seiten 12595-12602, XP55007311, ISSN: 0743-7463, DOI: 10.1021/la801770n
- NEETU SINGH ET AL: "Covalent Tethering of Functional Microgel Films onto Poly(ethylene terephthalate) Surfaces", BIOMACROMOLECULES, Bd. 8, Nr. 10, 1. Oktober 2007 (2007-10-01), Seiten 3271-3275, XP55007320, ISSN: 1525-7797, DOI: 10.1021/bm700516v
- LYNCH I ET AL: "Novel method to prepare morphologically rich polymeric surfaces for biomedical applications via phase separation and arrest of microgel particles", JOURNAL OF PHYSICAL CHEMISTRY. B (ONLINE), AMERICAN CHEMICAL SOCIETY, COLUMBUS, OH, US, Bd. 110, Nr. 30, 3. August 2006 (2006-08-03), Seiten 14581-14589, XP002604261, ISSN: 1520-5207, DOI: 10.1021/JP061166A [gefunden am 2006-07-12]
- KIM M R ET AL: "Swelling induced detachment of chondrocytes using RGD-modified poly(N-isopropylacrylamide) hydrogel beads", BIOTECHNOLOGY PROGRESS, AMERICAN INSTITUTE OF CHEMICAL ENGINEERS, US, Bd. 18, Nr. 3, 1. März 2002 (2002-03-01), Seiten 495-500, XP009122732, ISSN: 8756-7938, DOI: 10.1021/BP020287Z [gefunden am 2002-06-07]
- MICHAEL J. SERPE ET AL: "Doxorubicin Uptake and Release from Microgel Thin Films", BIOMACROMOLECULES, Bd. 6, Nr. 1, 1. Januar 2005 (2005-01-01), Seiten 408-413, XP55016156, ISSN: 1525-7797, DOI: 10.1021/bm049455x
- AIPING ZHU ET AL: "The synthesis and characterization of polymerizable and biocompatibleN-maleic acyl-chitosan", JOURNAL OF BIOMEDICAL MATERIALS RESEARCH PART B: APPLIED BIOMATERIALS, Bd. 85B, Nr. 2, 1. Januar 2008 (2008-01-01), Seiten 489-495, XP55015892, ISSN: 1552-4973, DOI: 10.1002/jbm.b.30970
- NAGASE K ET AL: "Temperature-responsive intelligent interfaces for biomolecular separation and cell sheet engineering", JOURNAL OF THE ROYAL SOCIETY. INTERFACE, THE ROYAL SOCIETY, LONDON, GB, Bd. 6, Nr. SUPPL. 3, 6. Juni 2009 (2009-06-06), Seiten S293-S309, XP002604260, ISSN: 1742-5689, DOI: 10.1098/RSIF.2008.0499.FOCUS [gefunden am 2009-03-25]
- RECUM VON H A ET AL: "NOVEL THERMALLY REVERSIBLE HYDROGEL AS DETACHABLE CELL CULTURE SUBSTRATE", JOURNAL OF BIOMEDICAL MATERIALS RESEARCH, WILEY, NEW YORK, NY, US, Bd. 40, Nr. 4, 15. Juni 1998 (1998-06-15), Seiten 631-639, XP001164210, ISSN: 0021-9304, DOI: 10.1002/(SICI)1097-4636(19980615)40:4<631: :AID-JBM15>3.0.CO;2-I
- TIANTIAN GAN ET AL: "In Situ Gelation of P(NIPAM-HEMA) Microgel Dispersion and Its Applications as Injectable 3D Cell Scaffold", BIOMACROMOLECULES, Bd. 10, Nr. 6, 8. Juni 2009 (2009-06-08), Seiten 1410-1415, XP55015891, ISSN: 1525-7797, DOI: 10.1021/bm900022m
- SAUNDERS B R ET AL: "Microgels: From responsive polymer colloids to biomaterials", ADVANCES IN COLLOID AND INTERFACE SCIENCE, ELSEVIER, NL, Bd. 147-148, 1. März 2009 (2009-03-01), Seiten 251-262, XP025994909, ISSN: 0001-8686, DOI: 10.1016/J.CIS.2008.08.008 [gefunden am 2008-08-24]
- OLIVEIRA DE W ET AL: "HYDROGELS FROM POLYSACCHARIDES-I. CELLULOSE BEADS FOR CHROMATOGRAPHIC SUPPORT", GLASS SCIENCE AND TECHNOLOGY, DEUTSCHE GLASTECHNISCHE GESELLSCHAFT, OFFENBACH, DE, Bd. 60, Nr. 1, 4. April 1996 (1996-04-04), Seiten 63-73, XP000584622, ISSN: 0946-7475
- DE OLIVEIRA W ET AL: "Hydrogels from polysaccharides. II. Beads with cellulose derivatives", JOURNAL OF APPLIED POLYMER SCIENCE, JOHN WILEY & SONS, INC, US, Bd. 61, Nr. 1, 1. Januar 1996 (1996-01-01) , Seiten 81-86, XP002641656, ISSN: 0021-8995
- OEZYUEREK Z ET AL: "Sulfated glyco-block copolymers with specific receptor and growth factor binding to support cell adhesion and proliferation", BIOMATERIALS, ELSEVIER SCIENCE PUBLISHERS BV., BARKING, GB, Bd. 30, Nr. 6, 1. Februar 2009 (2009-02-01), Seiten 1026-1035, XP025840047, ISSN: 0142-9612, DOI: 10.1016/J.BIOMATERIALS.2008.11.005 [gefunden am 2008-12-06]
- Xinqiao Jia ET AL: "Hyaluronic Acid-Based Microgels and Microgel Networks for Vocal Fold Regeneration", Biomacromolecules, 1. Dezember 2006 (2006-12-01), Seiten 3336-3344, XP55015967, Gefunden im Internet: URL:http://pubs.acs.org/doi/pdf/10.1021/bm 0604956 [gefunden am 2012-01-09]
- ZHANG Y ET AL: "Preparation of AgCl-polyacrylamide composite microspheres via combination of a polymer microgel template method and a reverse micelle technique", JOURNAL OF COLLOID AND INTERFACE SCIENCE, ACADEMIC PRESS, NEW YORK, NY, US, Bd. 300, Nr. 1, 1. August 2006 (2006-08-01), Seiten 210-218, XP024909375, ISSN: 0021-9797, DOI: 10.1016/J.JCIS.2006.03.068 [gefunden am 2006-08-01]
- RUBIO RETAMA J ET AL: "Biosensors based on acrylic microgels - A comparative study of immobilized glucose oxidase and tyrosinase", BIOSENSORS AND BIOELECTRONICS, ELSEVIER BV, NL, Bd. 20, Nr. 11, 15. Mai 2005 (2005-05-15), Seiten 2268-2275, XP004851721, ISSN: 0956-5663, DOI: 10.1016/J.BIOS.2004.10.011
- RETAMA J R ET AL: "Microstructural modifications induced by the entrapped glucose oxidase in cross-linked polyacrylamide microgels used as glucose sensors", BIOMATERIALS, ELSEVIER SCIENCE PUBLISHERS BV., BARKING, GB, Bd. 24, Nr. 17, 1. August 2003 (2003-08-01), Seiten 2965-2973, XP004424111, ISSN: 0142-9612, DOI: 10.1016/S0142-9612(03)00095-4
- ANDRIJ PICH ET AL: "Temperature Sensitive Hybrid Microgels with Magnetic Properties", LANGMUIR: THE ACS JOURNAL OF SURFACES AND COLLOIDS, AMERICAN CHEMICAL SOCIETY, WASHINGTON, DC, USA, Bd. 20, 23. November 2004 (2004-11-23), Seiten 10706-10711, XP002636554, ISSN: 0743-7463, DOI: 10.1021/LA040084F [gefunden am 2004-10-20]
- YANAN DU ET AL: "Surface-directed assembly of cell-laden microgels", BIOTECHNOLOGY AND BIOENGINEERING, Bd. 105, Nr. 3, 15. Februar 2010 (2010-02-15), Seiten 655-662, XP55015888, ISSN: 0006-3592, DOI: 10.1002/bit.22552
- EBARA M ET AL: "The effect of extensible PEG tethers on shielding between grafted thermo-responsive polymer chains and integrin-RGD binding", BIOMATERIALS, ELSEVIER SCIENCE PUBLISHERS BV., BARKING, GB, Bd. 29, Nr. 27, 1. September 2008 (2008-09-01), Seiten 3650-3655, XP022849893, ISSN: 0142-9612, DOI: 10.1016/J.BIOMATERIALS.2008.05.030 [gefunden am 2008-06-25]
- HERSEL U ET AL: "RGD modified polymers: biomaterials for stimulated cell adhesion and beyond", BIOMATERIALS, ELSEVIER SCIENCE PUBLISHERS BV., BARKING, GB, Bd. 24, Nr. 24, 1. November 2003 (2003-11-01), Seiten 4385-4415, XP004446082, ISSN: 0142-9612, DOI: 10.1016/S0142-9612(03)00343-0
- RANEE A STILE AND KEVIN E HEALY: "Thermo-responsive peptide-modified hydrogels for tissue regeneration", BIOMACROMOLECULES, ACS, US, Bd. 2, Nr. 1, 8. Februar 2001 (2001-02-08) , Seiten 185-194, XP009140533, ISSN: 1525-7797, DOI: 10.1021/BM0000945
- Andrew Lyon ET AL: "Thermoresponsive microgel-based materials", Chemical Society Reviews, 4. Februar 2009 (2009-02-04), Seiten 865-874, XP55016143, DOI: 10.1039/b715522k Gefunden im Internet: URL:http://pubs.rsc.org/en/content/article pdf/2009/cs/b715522k [gefunden am 2012-01-11]
- BOYKO V ET AL: "Thermo-sensitive poly(N-vinylcaprolactam-co-acetoacetoxyeth yl methacrylate) microgels: 1-synthesis and characterization", POLYMER, ELSEVIER SCIENCE PUBLISHERS B.V, GB, Bd. 44, Nr. 26, 1. Dezember 2003 (2003-12-01), Seiten 7821-7827, XP027140896, ISSN: 0032-3861 [gefunden am 2003-11-19]
- COLE M A ET AL: "Stimuli-responsive interfaces and systems for the control of protein-surface and cell-surface interactions", BIOMATERIALS, ELSEVIER SCIENCE PUBLISHERS BV., BARKING, GB, Bd. 30, Nr. 9, 1. März 2009 (2009-03-01), Seiten 1827-1850, XP025928051, ISSN: 0142-9612, DOI: 10.1016/J.BIOMATERIALS.2008.12.026 [gefunden am 2009-01-13]
- Yoshikatsu Akiyama ET AL: "Ultrathin Poly( N -isopropylacrylamide) Grafted Layer on Polystyrene Surfaces for Cell Adhesion/Detachment Control", Langmuir, vol. 20, no. 13, 1 June 2004 (2004-06-01), pages 5506-5511, XP55188328, ISSN: 0743-7463, DOI: 10.1021/la036139f

## Beschreibung

Die Erfindung betrifft ein thermoresponsives Substrat zur Aufnahme biologischer Zellen, insbesondere ein Substrat, dessen Oberflächeneigenschaften in Abhängigkeit von der Temperatur veränderlich sind. Des Weiteren betrifft die Erfindung ein Verfahren zur Herstellung eines derartigen Substrats, insbesondere ein Verfahren zur Aufbringung von thermoresponsivem Polymermaterial auf einen Substratkörper. Des Weiteren betrifft die Erfindung ein Verfahren zur Kultivierung biologischer Zellen auf einem thermoresponsiven Substrat. Anwendungen der Erfindung sind bei der in-vitro-Kultivierung biologischer Zellen gegeben.

Es ist allgemein bekannt, lebende biologische Zellen auf Substraten außerhalb eines Organismus zu kultivieren (*in-vitro*-Kultivierung). Ein zur Kultivierung vorgesehenes Substrat (Kultivierungssubstrat) besitzt typischerweise einen festen Substratkörper, z.B. aus Glas oder Kunststoff, dessen Oberfläche (Trägerfläche) funktionalisiert ist. Mit der Funktionalisierung, die z.B. eine Plasmabehandlung, eine Beschichtung mit Proteinen (wie z.B. Fibronektin, Kollagen) oder eine Beschichtung mit Polymeren (wie z.B. Polylysin) umfasst, wird eine Wechselwirkung der Zellen mit der Oberfläche beeinflusst. Es besteht ein Interesse an Kultivierungssubstraten, die eine gezielte Manipulation der biologischen Zellen und insbesondere eine Beeinflussung z.B. der Adhäsion, Migration, Proliferation, Differenzierung oder Zelltransformation (Bildung von Tumorzellen) ermöglichen. Ein grundsätzliches Problem stellt insbesondere die schonende Ablösung der Zellen von den Oberflächen dar. Dies geschieht in der Regel über eine Enzymbehandlung (Trypsinierung), die zu Zellschädigungen und Zellverlusten führen kann.

Durch Experimente wurde festgestellt, dass Eigenschaften biologischer Zellen durch die Härte der Oberfläche des Kultivierungssubstrats beeinflusst werden können. Beispielsweise führten Härtevariationen, die durch sequenzielle Beschichtungen mit Polyacrylamid und Biomolekülen erzielt wurden, zu verschiedenen Differenzierungen von mesenchymalen Stammzellen (siehe Discher et al. in "Cell" 126 (2006) 677-689). Des Weiteren ist bekannt, dass die Adhäsion biologischer Zellen von der Härte der Substratoberfläche abhängig ist.

Des weiteren sind thermoresponsive Polymere bekannt. Ein thermoresponsives Polymer zeichnet sich dadurch aus, dass es eine Schalttemperatur (LCST, "lower critical solution temperature") in wässrigen Medien aufweist. Wässrige Medien sind z. B. reines Wasser, kommerziell erhältliche Pufferlösungen, Zellkulturmedien oder Mischungen von Wasser mit organischen Lösungsmitteln. Unterhalb der Schalttemperatur sind wässrige Lösungen thermoresponsiver Polymere einphasig, darüber zweiphasig. Wenn thermoresponsive Polymere an Oberflächen immobilisiert werden, so vollziehen sie in wässrigen Medien bei Überschreiten der Schalttemperatur einen Phasenübergang (Konformationsübergang): sie sind unterhalb der Schalttemperatur stärker hydratisiert als oberhalb.

Es wurde festgestellt, dass die Adhäsion auf Substraten, die mit dem thermoresponsiven (thermosensitiven) Polymer Poly-(N-isopropyl-acrylamid) ("PNIPam") oder Derivaten davon beschichtet sind und die temperaturabhängige Hydratisierung aufweisen, gezielt in Abhängigkeit von der Temperatur beeinflusst werden kann (siehe N. Yamada et al. in "Makromol. Chem." 11 (1990) 571; C. Williams et al. in "Adv. Mater." 21 (2009) 2161-2164, O. Ernst et al. in "Lab Chip" 7 (2007) 1322). Diese Eigenschaft wurde auch an Polyethylenglykol (PEG)-basierten Polymeren gezeigt (siehe E. Wischerhoff et al. in "Angew. Chem." 47 (2008) 5666).

Herkömmliche Substrate, deren Oberflächen mit thermoresponsiven Polymeren beschichtet sind (z. B. WO 2004/011669), können sowohl in Bezug auf die Herstellung der Beschichtung als auch in Bezug auf die Eignung für die Zellkultivierung Nachteile haben. So erfordert die Herstellung eines mit einem thermoresponsiven Polymer beschichteten Substrats mehrere aufwendige Prozessschritte, die mit einem kostspieligen apparativen Aufbau realisiert werden. Des Weiteren besteht nur eine beschränkte Variabilität der Polymerzusammensetzung. Beispielsweise kann das thermische Ansprechverhalten des thermoresponsiven Polymers sich verändern oder verschwinden, wenn dem Polymer eine zweite Polymerkomponente zugesetzt wird. Daher besteht nur eine beschränkte Flexibilität hinsichtlich der Einführung einer weiteren Funktionalisierung eines mit einem thermoresponsiven Polymer beschichteten Substrats.

Zur Herstellung von mit thermoresponsiven Polymeren beschichteten Substraten wurden in der Praxis verschiedene Protokolle zur Funktionalisierung des Substratkörpers entwickelt, wie z.B. Reaktionen mit Silanen, eine Plasmabehandlung oder eine chemische Behandlung. Dabei werden an der Oberfläche des Substratkörpers funktionelle Gruppen, wie z.B. -NH₂, -COOH oder Epoxide bereitgestellt, die komplementär funktionalisierten Molekülen, wie insbesondere den thermoresponsiven Polymeren eine kovalente Anbindung ermöglichen. Dabei hat sich eine beschränkte Reproduzierbarkeit und Steuerbarkeit der Funktionalisierung insbesondere in Bezug auf die Anbindungsdichte und Homogenität, sowie die Beschränkung auf spezielle Substratmaterialien und chemische Substanzen und eine Beschränkung auf harte, planare Substratkörper als nachteilig erwiesen. Die Herstellung einer Oberfläche mit definierten Mischungen verschiedener Moleküle ist nur in speziellen Ausnahmefällen und mit hohem Aufwand möglich.

Für die Kultivierung biologischer Zellen hat sich insbesondere die folgende Eigenschaft thermoresponsiver Polymere als nachteilig erwiesen. Allgemein ist ein thermoresponsives Polymer ein Polymer, das in Abhängigkeit von der Temperatur einen physikalischen Phasenübergang durchläuft, bei dem z.B. eine Umordnung von Polymerketten erfolgt. Während der Phasenübergang in einer flüssigen Lösung in einem Temperaturbereich von wenigen °C scharf definiert ist, zeichnen sich schichtförmig immobilisierte thermoresponsive Polymere durch ein breites Temperaturprofil des Phasenübergangs aus. So wurde gefunden, dass für bestimmte Typen adhärenter Zellen eine Abkühlung von 37 °C auf Temperaturen unterhalb von 20 °C von bis zu einer Stunde erforderlich sind, um die Adhäsion von der Oberfläche des Substrats zu lösen (siehe "Application Notes" für die PNIPam-beschichteten UpCell-Kultivierungssubstrate des Herstellers Nunc). Eine derartige Abkühlung über diesen Zeitraum ist jedoch wegen der damit verbundenen möglichen Beeinflussung der Zellfunktion unerwünscht. Des Weiteren wurde in der Praxis festgestellt, dass thermoresponsive Polymerschichten für verschiedene Zelllinien, wie z.B. MCF7-Tumorzellen oder MG63 Osteoblasten-Zellen, unzureichend wirksam sein können.

Herkömmliche Techniken zeichnen sich ferner durch Nachteile bei der Kultivierung mit so genannten Kokulturen aus. Da ein zu kultivierender Zelltyp zum Wachstum oder zur Aufrechterhaltung der Vitalität im adhärenten Zustand Botenstoffe (parakrine Faktoren) von anderen Zellen benötigt, müssen häufig die Kultivierung, das Wachstum oder manipulative oder analytische Prozesse von adhärenten Zellen in der Kokultur gemeinsam durchgeführt werden (z.B. Stammzellen und Feederzellen oder Melanozyten und Keratinozyten). Für die anschließende Trennung der Zellen stehen bisher nur Verfahren zur Verfügung, die auf einer Zelltrennung in flüssigen Zellsuspensionen basieren. Hierzu müssen die Zellen vom Substrat abgelöst und in eine Trennvorrichtung (Durchfluss-Zytometer) überführt werden, was wegen des Zeit- und Präparationsaufwandes und der geringen Ausbeute erhebliche Nachteile hat. Insbesondere für Proben mit Zellzahlen unterhalb von 10⁵ Zellen ist die herkömmliche Zelltrennung nicht durchführbar, da bei der Bildung der Suspension und der Trennung im Durchfluss-Zytometer übermäßig viele Zellen verloren gehen.

Die Aufgabe der Erfindung ist es, ein verbessertes thermoresponsives Substrat zur Aufnahme biologischer Zellen bereitzustellen, mit dem Nachteile der herkömmlichen Technik überwunden werden. Die Aufgabe der Erfindung ist es insbesondere, ein verbessertes thermoresponsives Substrat bereitzustellen, das sich durch eine einfache Herstellung, eine hohe Flexibilität bei der Einstellung von Oberflächeneigenschaften, eine erweiterte Funktionalisierungsfähigkeit, eine Eignung für eine erweiterte Zahl von Zelltypen und/oder eine Eignung für eine schonende Zellkultivierung und Adhäsionssteuerung mit geringen Temperaturunterschieden auszeichnet. Eine weitere Aufgabe der Erfindung ist es, ein verbessertes Verfahren zur Herstellung eines thermoresponsiven Substrats, insbesondere zur Aufnahme biologischer Zellen, bereitzustellen, mit dem Nachteile herkömmlicher Verfahren zur Substratherstellung überwunden werden. Eine weitere Aufgabe der Erfindung ist es, ein verbessertes Kultivierungsverfahren unter Verwendung eines thermoresponsiven Substrats bereitzustellen, mit dem Nachteile und Beschränkungen herkömmlicher Kultivierungsverfahren überwunden werden.

Diese Aufgaben werden durch ein Substrat und Verfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt der Erfindung wird ein Substrat, insbesondere zur Aufnahme biologischer Zellen, wie in Anspruch 1 definiert, bereitgestellt. Gemäß einem zweiten Gesichtspunkt der Erfindung wird ein Verfahren zur Herstellung des erfindungsgemäßen Substrats wie in Anspruch 15 beschrieben, bereitgestellt.

Gemäß einem dritten Gesichtspunkt der Erfindung wird ein Verfahren zur Kultivierung biologischer Zellen auf dem erfindungsgemäßen Substrat bereitgestellt, bei dem die biologischen Zellen auf dem Substrat in Kontakt mit den freiliegenden thermoresponsiven Mikrogelen angeordnet werden. Erfindungsgemäß werden Kultivierungsbedingungen für die Zellen auf dem Substrat derart eingestellt, dass die Zellen einer zerstörungsfreien Ablösung (Abtrennung vom Substrat), einem Wachstum, einer Differenzierung und/oder einer Zellwanderung unterzogen werden.

Die erfindungsgemäße Bereitstellung eines Kultivierungssubstrats mit thermoresponsiven Mikrogelen hat eine Reihe von Vorteilen in Bezug auf die Einstellung physikalischer und/oder chemischer Oberflächeneigenschaften, die gezielte Veränderung von Oberflächeneigenschaften, die Herstellung des Substrats und die Schaffung neuer Anwendungen oder Funktionen von Kultivierungssubstraten. Die Erfinder haben festgestellt, dass der Phasenübergang der thermoresponsiven Mikrogele in einem engen Temperaturbereich erfolgt, welcher mit dem schmalen Temperaturprofil des Phasenübergangs gelöster thermoresponsiver Polymere vergleichbar ist. Breite Temperaturprofile über Intervalle von 20 °C bis 30 °C, wie sie bei herkömmlichen, isotropen Polymerschichten auftreten, werden erfindungsgemäß vermieden.

Die Erfinder haben festgestellt, dass sich der Phasenübergang durch eine Änderung eines Festigkeitsparameters des thermoresponsiven Polymers (z. B. Härte, plastische oder elastische Deformierbarkeit, insbesondere Young'scher Elastizitätsmodul) auszeichnet. Mit dem Festigkeitsparameter ändert sich die Adhäsion von Zellen unter- und oberhalb einer kritischen Temperatur des Phasenübergangs (Schalttemperatur des Polymers). Gleichzeitig ändert sich der Wassergehalt des thermoresponsiven Polymers. Im Ergebnis wird die Adhäsion der Zellen beeinflusst. Die Einstellung der Adhäsion ist vorteilhafterweise mit größerer Zuverlässigkeit und Reproduzierbarkeit möglich als bei herkömmlichen Polymerschichten. Die Erfinder haben festgestellt, dass mit dem Phasenübergang der immobilisierten Mikrogele eine wesentlich erhöhte Anzahl von Oberflächenwechselwirkungen angeboten oder unterbrochen und damit die Zuverlässigkeit einer temperaturgesteuerten Freigabe von Zellen verbessert wird.

In Bezug auf die gezielte Veränderung von Oberflächeneigenschaften hat sich als besonders vorteilhaft erwiesen, dass erfindungsgemäße Substrate einer Funktionalisierung für die biologischen Zellen unterzogen werden können, ohne dass die thermoresponsiven Mikrogele ihr Ansprechverhalten verlieren. Vorteile für die Herstellung des erfindungsgemäßen Substrats ergeben sich aus der Stabilität der Partikeldispersion über Wochen oder Monate und der unmittelbaren Gebrauchsfertigkeit des Substrats nach der Beschichtung der Trägerfläche mit den Mikrogelen. Die Funktionalisierung thermoresponsiver Substrate liefert neue Anwendungen, z.B. für eine passive Steuerung einer Zellwanderung auf der Substratoberfläche oder eine gezielte Ablösung von Zellen in vorbestimmten Substratbereichen.

Das erfindungsgemäße Substrat ist ein Kultivierungssubstrat für biologische Zellen. Das Substrat ist zur Aufnahme biologischer Zellen und zur Bereitstellung von physiologischen Kultivierungsbedingungen konfiguriert. Das Substrat ist insbesondere zur Aufnahme der Zellen in einem flüssigen Kultivierungsmedium eingerichtet, d.h. die Trägerfläche ist geeignet, das Kultivierungsmedium aufzunehmen. Der Substratkörper kann aus einem festen Material hergestellt sein, das starr oder nachgiebig (biegsam) ist. Das Material des Substratkörpers ist vorzugsweise temperaturstabil und insbesondere nicht thermoresponsiv. Die Trägerfläche ist vorzugsweise eine ebene Fläche, kann jedoch alternativ gekrümmt gebildet sein.

Die thermoresponsiven Mikrogele enthalten ein Polymer, das den Phasenübergang in einem physiologischen Temperaturbereich aufweist. Der Phasenübergang, der insbesondere ein Volumenphasenübergang ist, erfolgt vorzugsweise bei einer Temperatur unterhalb von 40 °C, insbesondere unterhalb von 37 °C, z.B. unterhalb von 35 °C. Bevorzugt erfolgt der Phasenübergang bei einer Temperatur oberhalb von 10 °C, insbesondere oberhalb von 20 °C, z.B. oberhalb von 32 °C. Das Temperaturintervall, in dem der Phasenübergang erfolgt, ist vorzugsweise schmaler als 15 °C, insbesondere schmaler als 10 °C, wie z.B. 5 °C oder weniger.

Die thermoresponsiven Mikrogele sind vorzugsweise aus mindestens einem ungeladenen und nichtionisierbaren Polymer hergestellt. Besonders bevorzugt bestehen die Mikrogele zumindest an ihrer Oberfläche aus dem mindestens einen ungeladenen und nichtionisierbaren Polymer. Vorteilhafterweise werden damit unerwünschte Wechselwirkungen mit der Zelloberfläche minimiert.

Gemäß bevorzugten Ausführungsformen der Erfindung sind die thermoresponsiven Mikrogele aus mindestens einem der Polymere der folgenden Polymere oder Polymergruppen gebildet:
(1) Poly-(N-isopropylacrylamid),
(2) -X-(-CH₂-CR₁COO-R₂-)ₙ-(-CH₂-CR₁COO-R₃-)ₘ-R₄ oder ein Copolymere davon,
(3) -X- [(-CH₂-CR₁COO-R₂-)ₙ-(-CH₂-CR₁COO-R₃-)ₘ-R₄] oder ein Copolymere davon, wobei
   X eine Kopplungsgruppe zur Trägerfläche, R₁ = H oder CH₃, R₂/R₃ = aliphatische Kohlenwasserstoffketten mit mindestens einer Ethergruppe, vorzugsweise mit 1 bis 20 Ethergruppen (bevorzugt Polyethylenoxid), und R₄ -H, eine aliphatische Kohlenwasserstoffkette oder eine funktionelle Gruppe ist, wie z.B. -Halogen, -N₃, -thiocarbonyl, -(di)thiocarbamyl
(4) Homo- oder Copolymere der allgemeinen Struktur 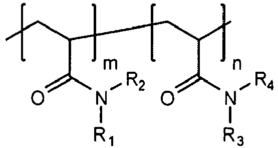 mit R₁, R₂, R₃ und R₄ = H oder Alkyl, bevorzugt R₁ = Isopropyl, R₂ = H, n = 0,
(5) Homo- oder Copolymere der allgemeinen Struktur 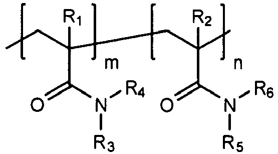
   mit R₁, R₂ = -H, -Alkyl, bevorzugt -H und -CH₃, besonders bevorzugt -H
   mit R₃, R₄, R₅ und R₆ = -H, -Alkyl, Alkenyl, Alkinyl, Aryl, bevorzugt -R₃ = -Isopropyl, -R₄ = -H, -R₅ und -R₆ = -C₂H₅, - CH₃ oder -H, besonders bevorzugt -R₃ = -Isopropyl und -R₄ = -H und m : n = 100 : 0 und / oder R₃, R₄, R₅ und 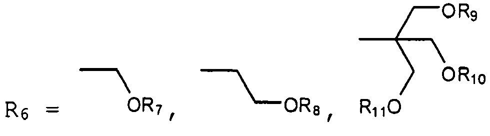 mit R₇, bis R₁₁ = -H, -Alkyl, Alkenyl, Alkinyl, Aryl, - Alkyloyl, mindestens ein R = H,
(6) Homo- oder Copolymere der allgemeinen Struktur 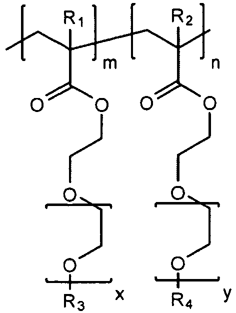 mit R₁, R₂ = H oder CH₃, R₃, R₄ = H oder Alkyl, x, y = 0 bis 20,
(7) Homo- und Copolymere der allgemeinen Struktur 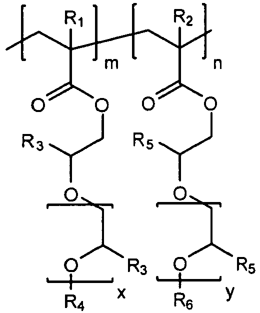
   mit R₁, R₂ = -H, -Alkyl, bevorzugt -H und -CH₃, besonders bevorzugt -CH₃,
   mit R₃, R₅ = -H, -Alkyl, Alkenyl, Alkinyl, Aryl, bevorzugt -H und -CH₃, besonders bevorzugt -H, wobei:
      wenn R₃, ≠ -H, dann R₅ = -H
   mit R₄, R₆ = -H, -Alkyl, Alkenyl, Alkinyl, Aryl, bevorzugt -H und / oder -CH₃, besonders bevorzugt -CH₃, wobei
      wenn R₃, R₅ = -H und R₄, R₆ = -CH₃, x = 1 und y = 7, 5, dann liegt m : n bevorzugt zwischen 95 : 5 und 90 : 10, besonders bevorzugt bei 93 : 7, und
   wenn R₃, R₅ = -H und R₄, R₆ = -CH₃, x = 1 und y = 4,5, dann liegt m : n bevorzugt zwischen 93 : 7 und 80 : 20, und besonders bevorzugt bei 85 : 15,
(8) Homo- und Copolymere der allgemeinen Struktur 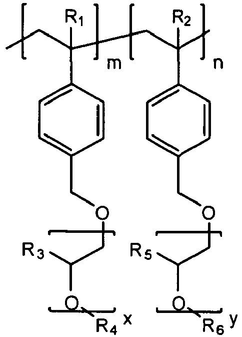
   mit R₁, R₂ = -H, -Alkyl, bevorzugt -H und -CH₃, besonders bevorzugt -H,
   mit R₃, R₅ = -H, -Alkyl, Alkenyl, Alkinyl, Aryl, bevorzugt -H und -CH₃, besonders bevorzugt -H, wobei
      wenn R₃, ≠ -H, dann R₅ = -H
   mit R₄, R₆ = -H, -Alkyl, Alkenyl, Alkinyl, Aryl, bevorzugt -H und / oder -CH₃, besonders bevorzugt -H, wobei
      wenn R₁, R₂ = -H, und R₄, R₆ = -H, x =3 und y = 4, dann liegt m : n bevorzugt zwischen 65 : 35 und 45 : 55, besonders bevorzugt zwischen 60 : 40 und 50 : 50,
(9) Homo- und Copolymere der allgemeinen Struktur 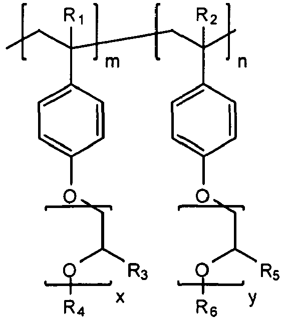 mit R₁, R₂ = -H, -Alkyl, bevorzugt -H und -CH₃, besonders bevorzugt -H, mit R₃, R₅ = -H, -Alkyl, Alkenyl, Alkinyl, Aryl, bevorzugt -H und -CH₃, besonders bevorzugt -H, wobei wenn R₃, ≠ -H, dann R₅ = -H, mit R₄, R₆ = -H, -Alkyl, Alkenyl, Alkinyl, Aryl, bevorzugt -H und / oder -CH₃, besonders bevorzugt -H,
(10) Homo- oder Copolymere der allgemeinen Struktur 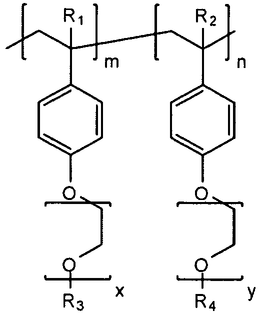 mit R₁, R₂ = H oder CH₃, R₃, R₄ = H oder Alkyl, x, y = 2 bis 20,
(11) Homo- oder Copolymere der allgemeinen Struktur 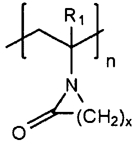 mit R₁ = H oder CH₃, x = 3 bis 5, Copolymere mit x = 3 und X > 3,
(12) Homo- oder Copolymere der allgemeinen Struktur 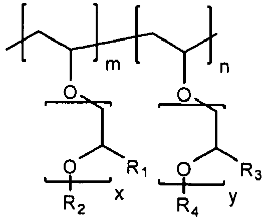 mit R₁, R₃ = -H, -Alkyl, Alkenyl, Alkinyl, Aryl, bevorzugt -H und -CH₃, besonders bevorzugt -H, wobei wenn R₃, ≠ -H, dann R₅ = -H, mit R₂, R₄ = -H, -Alkyl, Alkenyl, Alkinyl, Aryl, bevorzugt -H und / oder -CH₃, besonders bevorzugt -CH₃,
(13) Homo- oder Copolymere der allgemeinen Struktur 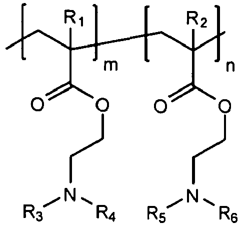 oder 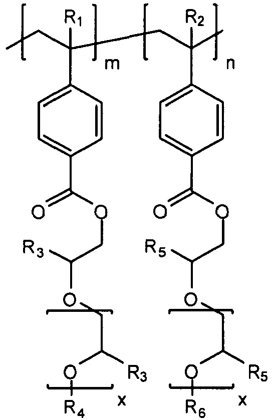
   mit R₁, R₂ = -H, -Alkyl, bevorzugt -H und -CH₃, besonders bevorzugt -H,
   mit R₃, R₅ = -H, -Alkyl, Alkenyl, Alkinyl, Aryl, bevorzugt -H und -CH₃, besonders bevorzugt -H, wobei
      wenn R₃, ≠ -H, dann R₅ = -H,
   mit R₄, R₆ = -H, -Alkyl, Alkenyl, Alkinyl, Aryl, bevorzugt -H und / oder -CH₃, besonders bevorzugt -H,
(14) Homo- oder Copolymere der allgemeinen Struktur 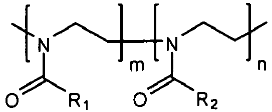 mit R₁, R₂ = -H, -Alkyl, Alkenyl, Alkinyl, Aryl,
(15) Homo- oder Copolymere der allgemeinen Struktur 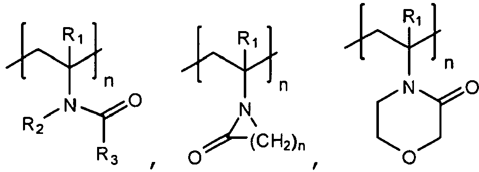 oder Copolymere der drei Elemente in Zusammensetzungen
   mit R₁ = -H, -Alkyl, bevorzugt -H und -CH₃, besonders bevorzugt -H,
   mit R₂, R₃ = -H, -Alkyl, Alkenyl, Alkinyl, Aryl mit 2 ≤ n ≤ 10, bevorzugt 3 ≤ n ≤ 6
(16) Homo- oder Copolymere der allgemeinen Struktur 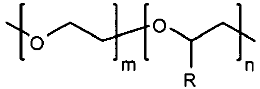 mit -R = -H, -Alkyl, Alkenyl, Alkinyl, Aryl, bevorzugt -R = - Alkyl, besonders bevorzugt -R = -CH₃,
(17) Homo- oder Copolymere der allgemeinen Struktur 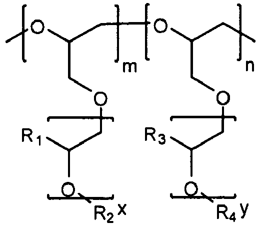
   mit R₁, R₃ = -H, -Alkyl, Alkenyl, Alkinyl, Aryl, bevorzugt -H und -CH₃, wobei
      wenn R₁, ≠ -H, dann R₃ = -H
   mit R₂, R₄ = -H, -Alkyl, Alkenyl, Alkinyl, Aryl, bevorzugt -H und / oder -CH₃,
(18) Homo- oder Copolymere der allgemeinen Struktur 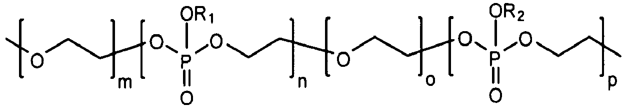 mit R₁, R₂ = -H, -Alkyl, Alkenyl, Alkinyl, Aryl,
(19) Homo- oder Copolymere der allgemeinen Struktur 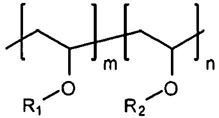 mit R₁, R₂ = -H, -Alkyl, Alkenyl, Alkinyl, Aryl, bevorzugt R₁ = -CH₃, n = 0,
(20) Homo- oder Copolymere der allgemeinen Struktur 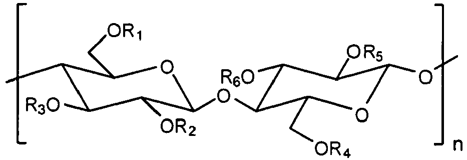 mit R₁ bis R₆ = -H, -Alkyl, Alkenyl, Alkinyl, Aryl, - Alkyloyl, mindestens zwei R = H,
(21) Homo- oder Copolymere analoger Struktur wie unter 19, basierend auf anderen Polysaccharidgerüsten als Cellulose,
(22) Homo- oder Copolymere mit elastin-ähnlichen Einheiten,
(23) Copolymere aller oben genannten Einheiten mit veränderten Monomeren.

Die genannten Copolymere können statistische, alternierende oder Blockcopolymere sein.

Bei den Polymeren gemäß (4) bis (25) beinhaltet mindestens eine terminale Einheit der Polymerhauptkette vorzugsweise eine Kopplungsgruppe zur Trägerfläche.

Die Trägerfläche kann die unmittelbare Oberfläche des Substratkörpers, z. B. aus Glas, Silizium oder Kunststoffen, wie Polystyrol, COP (Cyclo-Olefin Polymer), Polykarbonat, sein oder durch einen Metallfilm, z.B. aus Gold, Silber, Platin, Titan oder Chrom, auf dieser gebildet werden. In Abhängigkeit von der chemischen Zusammensetzung der Trägerfläche kann X eine funktionelle Gruppe sein, wie z.B. -SS-, -SH, -COOH, -NH2 (SS ist eine beidseits symmetrisch substituierte Disulfidgruppe). Vorzugsweise ist n+m > 10.

Diese Polymere haben sich in Bezug auf die Biokompatibilität und die Flexibilität bei der Einstellung physikalischer oder chemischer Oberflächeneigenschaften als besonders vorteilhaft erwiesen.

Die Mikrogele auf der Trägerfläche können sämtlich identisch aus einem einzigen Polymer gebildet sein. Wenn gemäß einer alternativen Variante die Mikrogele aus mindestens zwei verschiedenen Polymeren gebildet sind, können sich Vorteile bei der Einstellung der Oberflächeneigenschaften ergeben. Des Weiteren können in diesem Fall Mikrogele mit verschiedenen Zusammensetzungen auf der Trägerfläche angeordnet sein. Mikrogele mit verschiedenen Zusammensetzungen können z.B. nach Teilbereichen getrennt vorgesehen sein, um auf der Trägerfläche verschiedene Kultivierungsbedingungen bereitzustellen. Alternativ können die Mikrogele mit den verschiedenen Zusammensetzungen miteinander vermischt auf der Trägerfläche verteilt angeordnet sein. Alternativ oder zusätzlich können die Mikrogele verschiedene Durchmesser aufweisen. Beispielsweise können thermoresponsive Mikrogele mit verschiedenen Durchmessern getrennt in verschiedenen Teilbereichen der Trägerfläche fixiert oder miteinander vermischt über die Trägerfläche verteilt angeordnet sein.

Vorteilhafterweise können Mikrogele mit einem vorbestimmten Durchmesser der dispergierten, kolloidalen Teilchen hergestellt werden (siehe M. Andersson, S.L. Maunu, in "J. Poly. Sci." B 44 (2006) 3305; X. Wu et al. in "Coll. Poly. Sci." 272 (1994) 467). Dies ermöglicht, die Größe, oder, falls Partikel mit verschiedenen Durchmessern angeordnet werden, die verschiedenen Größen der thermoresponsiven Mikrogele gezielt einzustellen. Gemäß bevorzugten Ausführungsformen der Erfindung haben die thermoresponsiven Mikrogele einen Durchmesser von mindestens 10 nm, insbesondere mindestens 20 nm, besonders bevorzugt mindestens 50 nm, wie z.B. mindestens 100 nm. Die Obergrenze des Partikeldurchmessers beträgt vorzugsweise 50 µm. Besonders bevorzugt ist der Durchmesser der thermoresponsiven Mikrogele kleiner oder gleich 30 µm, insbesondere kleiner oder gleich 20 µm, wie z.B. 10 µm oder kleiner, z.B. kleiner als 1 µm.

Gemäß einer weiteren Variante können die thermoresponsiven Mikrogele eine Kern-Schale-Struktur aufweisen, wobei ein Kern entweder aus einem nicht-thermoresponsiven Material, insbesondere einem festen Trägerpartikel oder aus einem vernetzten thermoresponsiven Material bestehen kann. Vorzugsweise besteht ausschließlich die Schale aus dem thermoresponsiven Polymermaterial. Die Verwendung des festen Trägerpartikels, der z.B. aus anorganischem Glas, Metall, Keramik oder Kunststoff, insbesondere Polymethylmethacrylat oder Polystyrol, gebildet sein kann, kann Vorteile in Bezug auf die Bereitstellung einer bestimmten Mindesthärte der Oberfläche des erfindungsgemäßen Substrats haben. Vorzugsweise wird der Zusammenhalt der Kerne der thermoresponsiven Mikrogele durch Nebenvalenzwechselwirkungen (nicht kovalente Wechselwirkungen zwischen Molekülen wie van der Waals-Wechselwirkung, Wasserstoffbrückenbindung, hydrophobe Wechselwirkung) oder durch chemische Vernetzung bewirkt.

Weiterhin bieten Kern-Schale-Partikel die Möglichkeit, unvernetzte oder schwach vernetzte Polymerketten in ein Mikrogele zu integrieren, ohne den mechanischen Zusammenhalt des Partikels zu beeinträchtigen. Unvernetzte oder schwach vernetzte Mikrogele bieten den Vorteil, dass die thermoresponsiven Ketten sehr beweglich bleiben und so die Konformationsänderung beim Phasenübergang besser zum Tragen kommen kann. Sofern schwach vernetzte Partikel zum Einsatz kommen, soll die Vernetzungsdichte nicht mehr als 1 pro 20 Wiederholungseinheiten betragen und bevorzugt zwischen 1 pro 100 und 1 pro 500 Wiederholungseinheiten liegen. Um einen ausgeprägten thermoresponsiven Effekt zu erreichen, soll die Dicke der Schale mindestens 10 nm und höchstens 400 nm, bevorzugt zwischen 30 und 100 nm betragen. Generelle Beispiele für die Herstellung von Kern-Schale-Partikeln finden sich zum Beispiel Schuller in "Kolloid Z. Z. Polym." 211, 113-121 (1966), Fulda et al. in "Progr. Colloid Polym. Sci." 101, 178-183 (1996), oder Gao et al. in "Macromolecules" 39, 3154-3160 (2006).

Vorzugsweise ist die Dicke der Schicht aus thermoresponsiven Mikrogelen auf der Trägerfläche kleiner oder gleich dem Durchmesser der Mikrogele. Die Mikrogele bilden vorzugsweise zusammen mit Modulatorpartikeln eine Monolage. Es kann insbesondere eine geschlossene Monolage vorgesehen sein. Die Mikrogele können eine Sub-Monolage mit Lücken zwischen den Mikrogelen bilden.

Da die fixierten Mikrogelen eine abgeflachte Gestalt haben können, kann die Dicke der Schicht aus thermoresponsiven Mikrogelen kleiner als der Partikeldurchmesser sein. Mikrogele-Monolagen haben gegenüber Mehrfachlagen den Vorteil, dass die Adhäsion der biologischen Zellen mit größerer Zuverlässigkeit steuerbar ist.

Alternativ kann eine Schicht mehrere Lagen der thermoresponsiven Mikrogele enthalten. In diesem Fall können sich Vorteile durch eine größere Robustheit gegenüber Defekten bieten. Gemäß einer besonders bevorzugten Ausführungsform der Erfindung kann die Trägerfläche mit einem Haftvermittler ausgestattet sein. Als Haftvermittler kann jede Substanz verwendet werden, die zur Immobilisierung der Mikrogele geeignet und biokompatibel ist. Der Haftvermittler kann z.B eine kovalente Bindung mit der Trägerfläche und dem thermoresponsiven Polymer bilden. Des Weiteren können zur Immobilisierung der Polymere spezifische biologische Rezeptor-Ligand-Bindungen, wie z.B. die Bindung von Streptavidin und Biotin verwendet werden. Schließlich kann der Haftvermittler für eine Kopplung des Polymers durch eine unspezifische Wechselwirkung, wie z.B. eine Ladungs-Wechselwirkung, eine hydrophobe Wechselwirkung oder eine van-der-Waals-Wechselwirkung, ausgelegt sein. Mit diesen Wechselwirkungen wird das thermoresponsive Polymer auf der Trägerfläche temperaturunabhängig fest verankert. Die Verankerung der Partikel auf der Trägerfläche bleibt unabhängig vom Phasenübergang des thermoresponsiven Polymers bestehen.

Gemäß der Erfindung ist das Substrat mit mindestens einer Modulatorsubstanz, auf Modulatorpartikeln (5.1, 5.2) angeordnet, ausgestattet. Die mindestens eine Modulatorsubstanz ist auf der freiliegenden Oberfläche des Substrats, d.h. zwischen den thermoresponsiven Mikrogelen und/oder diese zumindest teilweise überdeckend angeordnet. Die Bereitstellung der mindestens einen Modulatorsubstanz ermöglicht vorteilhafterweise eine Funktionalisierung des Substrats. Somit stellt diese Ausführungsform der Erfindung einen wesentlichen Fortschritt gegenüber herkömmlichen Kultivierungssubstraten mit thermoresponsiven Polymeren dar, die für eine solche Funktionalisierung ungeeignet waren. Hierzu im Gegensatz ermöglicht die mindestens eine Modulatorsubstanz eine Beeinflussung der Kultivierungsbedingungen auf dem Substrat, ohne dass das Temperaturverhalten der Mikrogele beeinträchtigt wird.

Vorteilhafterweise können verschiedene Typen von Modulatorsubstanzen einzeln oder in Kombination vorgesehen sein. Beispielsweise können Modularsubstanzen vorgesehen, welche die Adhäsionsfähigkeit der biologischen Zellen steigern (adhäsionssteigernde Modulatorsubstanzen, zellanziehende Moleküle). Hierzu ist mindestens eine der folgenden Substanzen vorgesehen:
- Biomoleküle, wie z.B. Fibronektin, Kollagen, Laminin,
- adhäsionsvermittelnde Peptide, wie z.B. Peptide, welche die Aminosäurefolge RGD enthalten,
- synthetische Polymere, wie z.B. Poly-L-Lysin, Polystyrolsulfonat, Polyallylamin, Polyethylenimin.

Alternativ können Modulatorsubstanzen vorgesehen sein, welche die Adhäsionsfähigkeit der biologischen Zellen vermindern (adhäsionsmindernde Modulatorsubstanzen, zellabstoßende Molekülen). In diesem Fall wird mindestens eine der folgenden Substanzen als Modulatorsubstanz verwendet:
- Proteine, wie z.B. Rinderserumalbumin (bovine serum albumin, BSA),
- adhäsionsmindernde Peptide, wie z.B. Peptide mit hohem Anteil an Leucin und Isoleucin,
- synthetische Polymere, wie z.B. Polymere, die Ketten aus Polyethylenglycol ("PEG") enthalten, und
- Lipide.

Es können des Weiteren adhäsionssteigernde und adhäsionsmindernde Modulatorsubstanzen auf einem Substrat kombiniert vorgesehen sein. Beispielsweise können die Modulatorsubstanzen mit verschiedenen Wirkungen in verschiedenen Teilbereichen der Trägerfläche getrennt oder miteinander vermischt über die Trägerfläche verteilt angeordnet sein. Im letzteren Fall kann durch das Mischungsverhältnis von adhäsionssteigernden und adhäsionsmindernden Modulatorsubstanzen eine effektive Adhäsionsfähigkeit in der Oberfläche des Substrats eingestellt werden, wobei durch die gleichzeitige Bereitstellung der thermoresponsiven Mikrogele die temperaturabhängige Fixierung oder Ablösung von Zellen erhalten bleibt.

Gemäß einer weiteren Variante der Erfindung kann alternativ oder zusätzlich eine Modulatorsubstanz vorgesehen sein, die geeignet ist, in den biologischen Zellen zelluläre Reaktionen zu induzieren. Beispielsweise kann vorgesehen sein, dass eine Modularsubstanz an Oberflächenrezeptoren der Zellen anbindet, um die Reaktionen auszulösen. Für diese Funktion sind Substanzen, wie z.B. Proteine der extrazellulären Matrix (ECM) wie Fibronektin, Antikörper gegen Rezeptoren (EGFR), die Wachstumsfaktoren binden, oder Antikörper z.B. gegen CD 28 und CD 3 von T-Zellen (Aktivierung der Immunantwort) besonders bevorzugt vorgesehen.

Erfindungsgemäß erfolgt die Bereitstellung der mindestens einen Modulatorsubstanz durch Modulatorpartikel, die aus der mindestens einen Modulatorsubstanz bestehen oder mit dieser beschichtet sind und zwischen den thermoresponsiven Mikrogelen auf der Trägerfläche angeordnet sind. Vorteilhafterweise können die Modulatorpartikel dem Mikrogel zugesetzt und mit diesem auf die Trägerfläche aufgetragen werden.

Vorteilhafterweise kann gemäß einer weiteren Ausführungsform der Erfindung eine räumliche Modulation der Oberflächeneigenschaften des Substrats auf der Trägerfläche vorgesehen sein. Die Trägerfläche weist in mindestens zwei Teilbereichen verschiedene Oberflächeneigenschaften auf. Vorteilhafterweise können die Teilbereiche dadurch gebildet werden, dass mindestens eines von den thermoresponsiven Mikrogelen, dem Haftvermittler und der mindestens einen Modulatorsubstanz auf der Trägerfläche mit mindestens einem räumlichen Dichtegradienten angeordnet wird. Mindestens eine der genannten Komponenten der Oberfläche des Substrats ist mit einer räumlichen Dichte vorgesehen, die in mindestens einer Richtung entlang der Trägerfläche veränderlich ist. Der Dichtegradient kann stufenweise oder kontinuierlich gebildet sein. Die Bereitstellung des mindestens einen Dichtegradienten ermöglicht vorteilhafterweise, dass die Zellen entlang der Trägerfläche verschiedene Adhäsionsfähigkeiten, verschiedene Temperaturreaktionen, verschiedene zelluläre Reaktionen, wie z.B. verschiedene Differenzierungen, und/oder verschiedene Zellwanderungen zeigen.

Gemäß einer weiteren vorteilhaften Ausführungsform der Erfindung kann auf der Trägerfläche mindestens eine Kultivierungskavität vorgesehen sein. Die Kultivierungskavität ist ein über die Trägerfläche hinausragender Vorsprung, der die Trägerfläche partiell überdeckend gebildet ist. Die Kultivierungskavität umfasst z.B. die Gestalt eines einseitig offenen Hohlraums oder einer Tasche, und sie ist zur Aufnahme von mindestens einer biologischen Zelle eingerichtet. Mit der Kultivierungskavität werden räumliche Kultivierungsbedingungen nachgebildet, die bei der Kultivierung im Zellverband gegeben sind.

Vorteilhafterweise kann der mindestens eine Dichtegradient zur Funktionalisierung der Oberfläche des Substrats so gebildet sein, dass Zellen zu der Kultivierungskavität wandern, um dort einer weiteren Kultivierung und/oder Differenzierung unterzogen zu werden.

Das erfindungsgemäße Substrat ist für die Kultivierung biologischer Zellen ausgelegt. Hierzu ist der Substratkörper vorzugsweise ein Teil einer Kultivierungseinrichtung, wie z.B. eines Kultivierungsgefäßes oder einer Fluidikeinrichtung, in der Zellen kultivierbar sind, wie z.B. eines fluidischen Mikrosystems. Der Substratkörper kann mit der Kultivierungseinrichtung fest verbunden sein, z.B. den Boden des Kultivierungsgefäßes bilden, oder von der Kultivierungseinrichtung lösbar sein, z.B. ein in ein Kultivierungsgefäß einlegbares Teil darstellen.

Das erfindungsgemäße Verfahren zur Kultivierung biologischer Zellen kann mit einem oder mehreren der folgenden Verfahrensschritte ausgeführt werden. So kann eine Einstellung der Adhäsion der biologischen Zellen auf dem Substrat vorgesehen sein, indem die Temperatur des Substrats eingestellt wird. Die Temperatureinstellung kann global für das gesamte Substrat oder lokal für mindestens einen Teilbereich vorgesehen sein. Mit der Temperatureinstellung wird ein Festigkeitsparameter der Substratoberfläche beeinflusst. Des Weiteren kann eine Einstellung einer zelltypspezifischen Migration von mindestens einem Typ der biologischer Zellen vorgesehen sein, indem mindestens ein Zelltyp, z. B. mindestens ein Differenzierungstyp, entlang eines Dichtegradienten einer Modulatorsubstanz zur Migration angeregt wird. Des Weiteren kann eine Einstellung der Migration von mindestens einem Zelltyp mittels des Dichtegradienten der Modulatorsubstanz derart vorgesehen sein, dass die biologischen Zellen in eine Kultivierungskavität wandern.

Weitere Einzelheiten und Vorteile der Erfindung werden im Folgenden unter Bezug auf die beigefügten Zeichnungen erläutert. Es zeigen:
- Figur 1:: eine schematische Perspektivansicht einer ersten Ausführungsform des erfindungsgemäßen Substrats;
- Figur 2:: schematische Illustrationen des Phasenübergangs von thermoresponsiven Mikrogelen;
- Figur 3:: experimentelle Ergebnisse, die den Phasenübergang von thermoresponsiven Mikrogelen zeigen;
- Figur 4:: eine schematische Illustration eines thermoresponsiven Mikrogels mit Kern-Schale-Struktur;
- Figuren 5 und 6:: Anordnungsmöglichkeiten der Mikrogele ohne Modulatorpartikel;
- Figuren 7 bis 8:: weitere Ausführungsformen erfindungsgemäßer Substrate;
- Figur 9:: eine schematische Illustration einer Kultivierungseinrichtung, die mit dem erfindungsgemäßen Substrat ausgestattet ist;
- Figuren 10 bis 12:: schematische Illustrationen weiterer Ausführungsformen erfindungsgemäßer Substrate, die mit mindestens einer Modulatorsubstanz ausgestattet sind;
- Figur 13:: schematische Illustration eines nichterfindungsgemäßen Substrats, das mit einer nicht auf Modulatorpartikeln befindlichen Modulatorsubstanz ausgestattet ist;
- Figuren 14 bis 17:: schematische Illustrationen weiterer Ausführungsformen eines erfindungsgemäßen Substrats (Fig. 14) und nicht erfindungsgemäßer Substrate (Fig. 15-17) mit Dichtegradienten von Oberflächenkomponenten; und
- Figur 18:: eine schematische Illustration eines Substrats mit einer Kultivierungskavität.

Bevorzugte Ausführungsformen der Erfindung werden im Folgenden unter Bezug auf die Bereitstellung von thermoresponsiven Mikrogelen auf der Trägerfläche eines Kultivierungssubstrats und dessen optional vorgesehene Funktionalisierung beschrieben. Einzelheiten von Kultivierungsverfahren, insbesondere von Verfahren zur Handhabung biologischer Zellen und deren gezielte Beeinflussung, werden nicht beschrieben, da diese an sich aus dem Stand der Technik bekannt sind. Des Weiteren wird betont, dass die beigefügten Zeichnungen schematische, vergrößerte Illustrationen von Ausschnitten des erfindungsgemäßen Kultivierungssubstrats darstellen. Die Umsetzung der Erfindung in der Praxis ist nicht auf die Illustrationen beschränkt, sondern mit abgewandelten Formen, Größen und Zusammensetzungen des Substrats möglich.

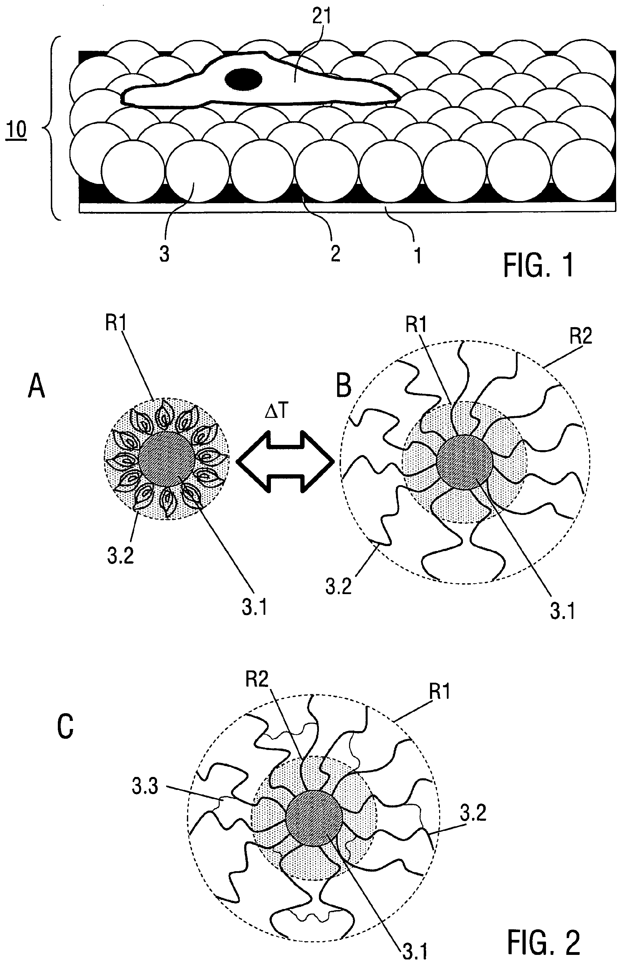

Figur 1 zeigt in schematischer Perspektivansicht eine erste Ausführungsform des erfindungsgemäßen Substrats 10 mit dem Substratkörper 1, auf dessen Oberseite (Trägerfläche 2) die thermoresponsiven Mikrogele 3 angeordnet sind. Der Substratkörper 1 besteht z. B. aus Metallen, wie Gold, Titan, Platin, Glas, Siliziumwafer oder Kunststoff, wie Polystyrol, COP, Polykarbonat, dessen Oberfläche die Trägerfläche 2 bildet. Die thermoresponsiven Mikrogele 3 sind aus den kolloidalen Bestandteilen eines thermoresponsiven Polymers gebildet. Abweichend von der schematisch illustrierten Kugelform können die thermoresponsiven Mikrogele 3 in der Praxis z.B. eine Halbkugelform oder eine in Abhängigkeit von den Beschichtungsbedingungen unregelmäßig deformierte Gestalt aufweisen.

Die thermoresponsiven Mikrogele 3, die z.B. aus PNIPam hergestellt sind, zeigen den in den Figuren 2A bis 2C schematisch illustrierten Phasenübergang. Die thermoresponsiven Mikrogele 3 umfassen einen vernetzten Kern 3.1, von dem radial nach außen abstehend Polymerketten 3.2 gebildet sind. Oberhalb einer kritischen Temperatur ("Lower Critical Solution Temperature", LCST, Schalttemperatur), die für Kultivierungsanwendungen typischerweise wenige °C, z. B. 2°C bis 10°C unterhalb von 37 °C gewählt ist, liegen die Polymerketten 3.2 in einem kollabierten Zustand vor (Figur 2A). Bei Abkühlung um eine vorbestimmte Temperaturdifferenz ΔT und Unterschreitung der kritischen Temperatur gehen die Polymerketten 3.2 in einen nicht-kollabierten (gequollenen) Zustand über (Figur 2B). Die Größe der thermoresponsiven Mikrogele wird mit einem hydrodynamischen Radius beschrieben, der im kollabierten Zustand (R1) geringer als im nicht-kollabierten Zustand (R2) ist. Im nicht-kollabierten Zustand, d.h. unterhalb der kritischen Temperatur können zwischen den Polymerketten 3.2 Kettenbrücken 3.3 bestehen bleiben (Figur 2C), was sich auf die mechanischen Deformationseigenschaften und damit auf die Adhäsionseigenschaften des Substrats 10 für biologische Zellen auswirkt.

In einem praktischen Beispiel ist der Radius R1 der kollabierten Partikel z.B. im Bereich von 2 nm bis 5 µm gewählt. Entsprechend kann ein Radius R2 im nicht-kollabierten Zustand z.B. im Bereich von 4 nm bis 10 µm erreicht werden (z. B. 200 nm bis 420 nm oder 300 nm bis 480 nm, siehe Wu et al. in "Coll. Poly. Sci." 272 (1994) 467; z. B. 92 nm bis 200 nm, von 42 nm bis 97 nm, von 29 nm bis 65 nm und von 19 nm bis 35 nm, siehe M. Andersson et al. in "J. Poly. Sci." B 44 (2006) 3305).

Um die thermoresponsiven Mikrogele 3 auf der Trägerfläche 2 zu immobilisieren, wird zunächst eine Mikrogel-Dispersion hergestellt, welche die Mikrogel-Partikel als kolloidale Teilchen enthält. Der Radius R1 wird durch die Reaktionsbedingungen bei der Herstellung des Mikrogels eingestellt. Die Mikrogel-Dispersion kann stabil gelagert werden.

Bei der Herstellung der Mikrogel-Dispersion werden vorzugsweise die folgenden Parameter der Partikel eingestellt:
- Polymerkettenlänge,
- Vernetzungsdichte (z.B. Bildung von Kettenbrücken),
- Partikelradius R1 im kollabierten Zustand,
- Partikelradius R2 im nicht-kollabierten Zustand,
- Elastizitätsmodul im kollabierten Zustand,
- Elastizitätsmodul im nicht-kollabierten Zustand, und
- (optional) radialer Gradient der Steifigkeit von innen nach außen.

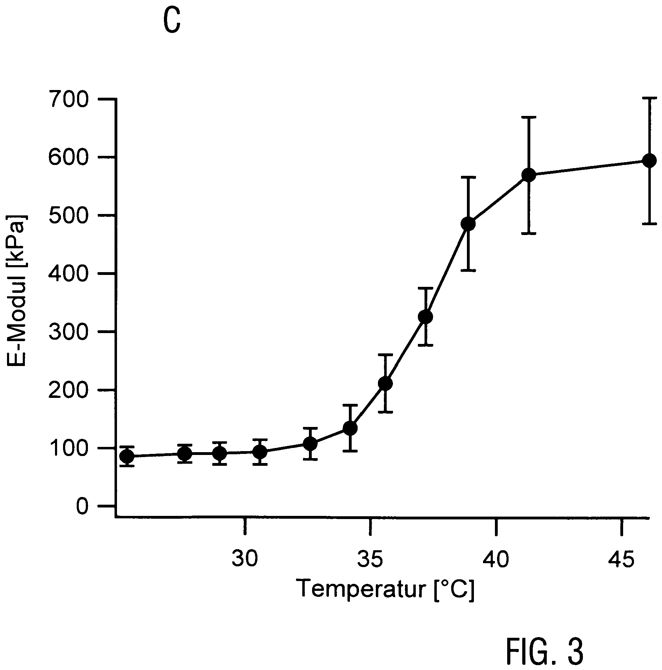

Die Mikrogel-Parameter werden in Abhängigkeit von der Anwendung des Substrats 10 gewählt. Obwohl mit den genannten Eigenschaften ein komplexer Parameterraum aufgespannt wird, ist die Auswahl der konkret zu verwendenden Parameter in Abhängigkeit von den zu kultivierenden Zellen und den zu realisierenden Kultivierungsbedingungen (geometrisch, physikalisch und chemisch) z.B. durch einfache Tests oder durch Verwendung von Tabellenwerten möglich. Die Erfinder haben festgestellt, dass zwischen der Adhäsion biologischer Zellen auf den thermoresponsiven Mikrogelen 3 und deren elastischen Eigenschaften eine starke Korrelation besteht, so dass durch die Auswahl insbesondere von elastischen Eigenschaften des Mikrogels eine Optimierung der Kultivierungsbedingungen möglich ist. So wurde z.B. festgestellt, dass bei einer Änderung des E-Moduls der Mikrogele von 600 kPa (oberhalb der LCST) auf 100 kPa (unterhalb der LCST) (siehe Fig. 3C) sehr gute Adhäsions- bzw. Zellablöseeigenschaften bestehen.

Zur Herstellung des Substrats 10 wird das Mikrogel auf die Trägerfläche 2 aufgebracht. Es wird eine an sich bekannte Depositionstechnik, wie z.B. Spin-Coating, Eintauchen, Aufsprühen, Stempeln oder Dispensieren, z.B. mit Nadeln oder Dispenserdüsen, verwendet. Die thermoresponsiven Mikrogele 3, die mit der Trägerfläche 2 in Kontakt kommen, bilden mit dieser z.B. eine kovalente Bindung. Anschließend wird die beschichtete Trägerfläche 2 gewaschen, z.B. mit Wasser, um die überschüssigen, nicht-gebundenen Teilchen abzutrennen. Die an der Trägerfläche 2 fixierten thermoresponsiven Mikrogele 3 können, wie in Figur 1 schematisch gezeigt, eine regelmäßige, dichte Packung oder alternativ eine unregelmäßige Packung mit Lücken bilden.

Anschließend kann ein Trocknen der mit den Mikrogelen 3 versehenen Trägerfläche 2 vorgesehen sein. Die Trocknung ist jedoch nicht zwingend vorgesehen. Alternativ kann unmittelbar nach dem Abwaschen eine zusätzliche Funktionalisierung oder die Kultivierung biologischer Zellen vorgesehen sein. Des Weiteren kann eine Sterilisierung der freiliegenden Oberfläche der thermoresponsiven Partikel, z.B. durch ionisierende Strahlung (Gammastrahlung) oder Begasung (z.B. mit Ethylenoxid) vorgesehen sein.

Wie in Figur 1 schematisch illustriert, ist mindestens eine biologische Zelle 21 auf der Oberfläche mit thermoresponsiven Mikrogelen 3 im kollabierten Zustand adhärent angeordnet. Durch eine Temperaturabsenkung kann der Phasenübergang der thermoresponsiven Mikrogele 3 in den nicht-kollabierten Zustand induziert werden, in dem die Härte der Oberfläche mit den thermoresponsiven Mikrogelen 3 im Vergleich zum kollabierten Zustand vermindert ist. Auf der Oberfläche mit der verminderten Härte hat die mindestens eine biologische Zelle 21 eine verminderte Adhäsionsfähigkeit, so dass sie durch das flüssige Kultivierungsmedium über dem Substrat (in Figur 1 nicht gezeigt) abgelöst, z. B. abgespült werden kann.

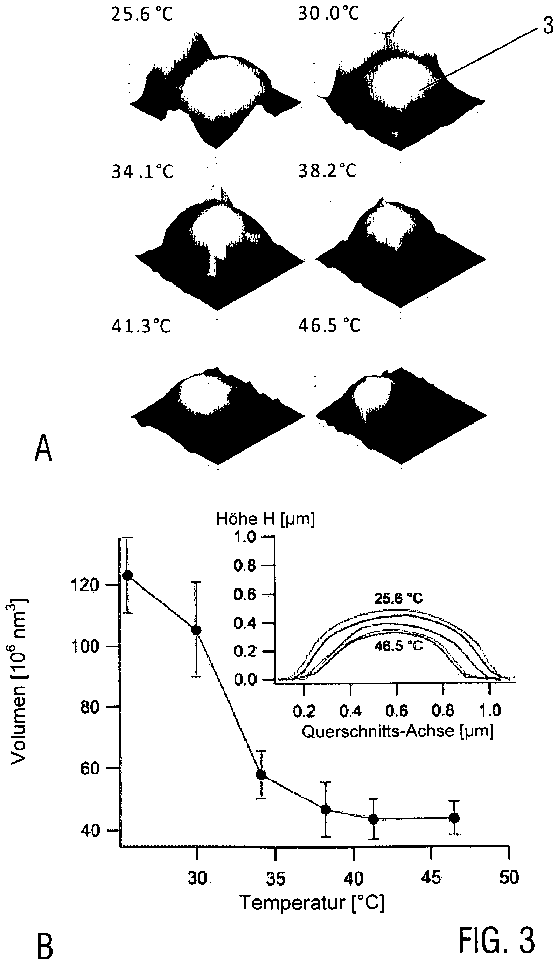

Experimentelle Ergebnisse, die den Phasenübergang von thermoresponsiven Mikrogelen 3 zeigen, sind beispielhaft in Figur 3 gezeigt. Figur 3A illustriert die mit einem Atomkraftmikroskop gemessene Topographie einzelner Mikrogele 3 (PNIPam) für verschiedene Temperaturen. Quellkurven der Mikrogele 3 im adsorbierten Zustand sind in Figur 3B gezeigt. Die kleine Graphik in Figur 3B zeigt mittlere Höhenprofile im Apex der Mikrogele 3 (Höhe H in Abhängigkeit von Durchmesserkoordinate, jeweils in µm). Schließlich illustriert Figur 3C die Temperaturabhängigkeit des Young' schen Elastizitäts-Moduls der Mikrogele, die aus Messungen mit dem Atomkraftmikroskop abgeleitet wurden. Während bei 37 °C das Elastizitäts-Modul grö-βer als 300 kPa, verringert sich das Elastizitäts-Modul bei 25 °C auf Werte unterhalb von 100 kPa. Gleichzeitig haben die Partikel bei der höheren Temperatur einen geringen Wassergehalt von rund 65 %, während bei 25 °C der Wassergehalt der Mikrogele rund 90% ist.

Die experimentellen Ergebnisse zeigen, dass das thermische Ansprechverhalten der Mikrogele 3, insbesondere das scharfe Temperaturprofil des Phasenübergangs, im adsorbierten Zustand mit dem flüssigen Zustand vergleichbar ist. Experimentelle Tests mit Maus-Fibroblasten haben ergeben, dass die Adhäsion der Fibroblasten auf der Oberfläche in dem Temperaturbereich, in dem der Phasenübergang mit der Änderung des Young-Moduls gemessen wurde, von einem adhärenten Zustand bei Temperaturen oberhalb des Phasenübergangs in einen nicht-adhärenten Zustand bei Temperaturen unterhalb des Phasenübergangs verstellt werden konnte. Oberhalb des Phasenübergangs, z. B. bei 37 °C haben die Zellen eine größere Kontaktfläche mit dem Substrat als bei Temperaturen unterhalb des Phasenübergangs.

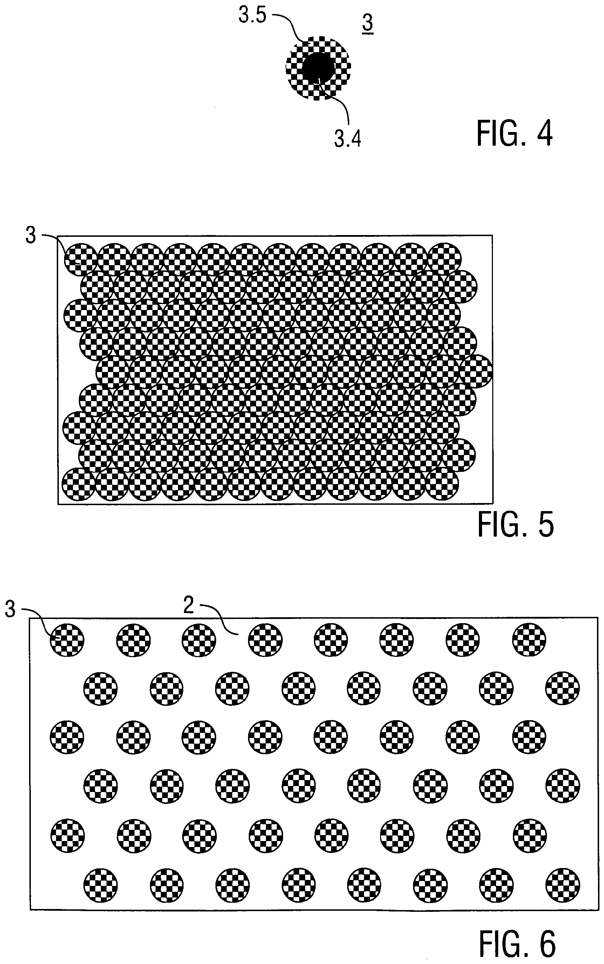

Die Figur 4 zeigt eine Variante der Erfindung, welche insbesondere in Abhängigkeit von der konkreten Kultivierungsaufgabe gewählt werden kann. So ist gemäß Figur 4 ein thermoresponsives Mikrogel 3 mit einer Kern-Schale-Struktur vorgesehen. Der Kern 3.4, z.B. aus Latex, ist unter den Kultivierungsbedingungen und insbesondere bei einer Temperaturänderung unveränderlich. Die Schale 3.5 wird durch das thermoresponsive Polymer, z.B. PNIPam gebildet. Die Herstellung einer Mikrogel-Dispersion zur Bildung von Partikeln mit Kern-Schale-Struktur ist an sich bekannt (siehe Hellweg et al. in "Langmuir" 20 (2004) 4330; Fernändez-Barbero et al. in "Phys Rev E 66" (2002) 051803/1-10). Die Fixierung von thermoresponsiven Mikrogelen 3 mit Kern-Schale-Struktur auf der Trägerfläche und die weitere Behandlung des Substrats erfolgt, wie dies oben unter Bezug auf Figur 1 beschrieben ist.

Die thermoresponsiven Mikrogele 3 können - ohne Modulatorpartikel - eine geschlossene Monolage (Figur 5; diese Variante ist nicht Gegenstand der Erfindung) oder eine von Lücken unterbrochene, nicht-geschlossene Monolage (Figur 6) auf der Trägerfläche 2 bilden. Die regelmäßige Anordnung der thermoresponsiven Mikrogele 3 gemäß Figur 5 kann durch Selbstorganisation (Bildung der dichtesten Packung) erzeugt werden. Bei der nicht-geschlossenen Schicht gemäß Figur 6 hingegen kann die regelmäßige Anordnung der thermoresponsiven Mikrogele 3 durch eine Vorbehandlung der Trägerfläche, z.B. mit lokal aufgebrachten Haftvermittler-Inseln erreicht werden. Abweichend von den Figuren 5 und 6 können die thermoresponsiven Mikrogele 3 unregelmäßige Anordnungen auf der Trägerfläche 2 bilden.

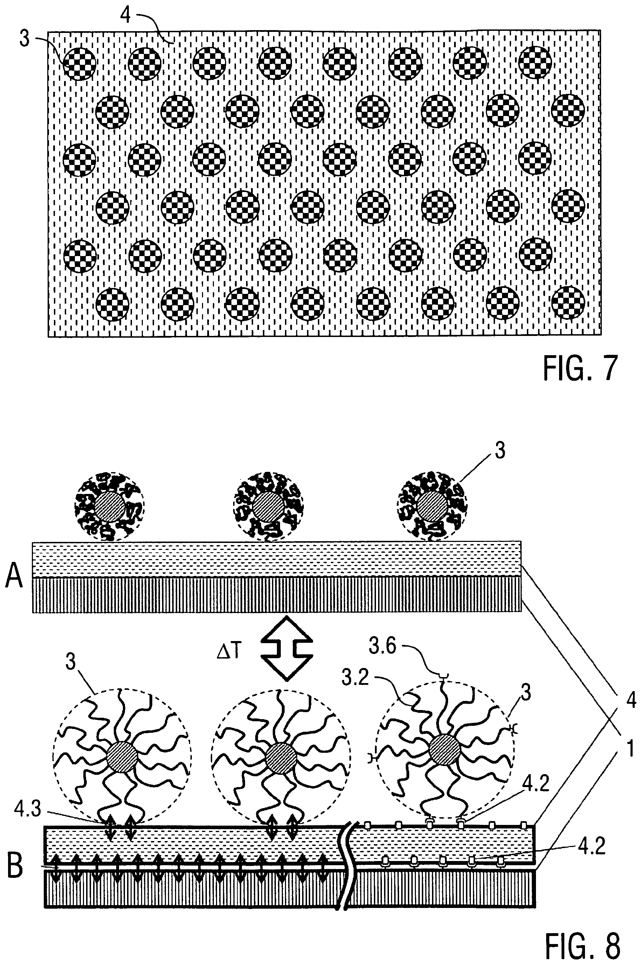

Die Figuren 7 und 8 illustrieren schematisch, dass die Verankerung der thermoresponsiven Mikrogele 3 auf der Trägerfläche 2 verbessert werden kann, wenn auf dieser ein Haftvermittler 4 angeordnet ist. Der Haftvermittler 4 kann die Trägerfläche 2 komplett bedecken (Figur 7) und so die für die Fixierung der thermoresponsiven Mikrogele 3 freiliegende, modifizierte Trägerfläche bilden. Figur 8 zeigt einen Ausschnitt des erfindungsgemäßen Substrats 10 mit den thermoresponsiven Mikrogelen 3 im kollabierten Zustand (Figur 8A) oberhalb der kritischen Temperatur und im nicht-kollabierten Zustand (Figur 8B) unterhalb der kritischen Temperatur. Figur 8B illustriert des Weiteren schematisch die zwischen dem Haftvermittler 4 mit einerseits dem Substratkörper 1 und andererseits den thermoresponsiven Partikeln 3 vorgesehenen Bindungsvarianten. So können an den freien Enden der Polymerketten 3.2 der thermoresponsiven Mikrogele 3 Bindungsplätze 3.6 für eine kovalente oder biospezifische Bindung 4.1 mit dem Haftvermittler 4 vorgesehen sein. Die Bindungsplätze 3.6 können bei der Herstellung des Mikrogels gebildet werden. Die kovalenten Bindungen basieren auf z.B. Epoxid-, Carboxy-, Amino-, Hydrazid-, Thiol- oder Maleimid-Verbindungen an den Enden der Polymerketten 3.2. Zur Bildung der kovalenten oder biospezifische Bindung 4.1 ist die Haftvermittler-Schicht 4 entsprechend mit Bindungsplätzen 4.2 ausgestattet, die mit den Bindungsplätzen 3.6 der thermoresponsiven Mikrogele 3 reagieren. Gleichzeitig bilden die Bindungsplätze 4.2 mit dem Substratkörper 1 kovalente oder biospezifische Bindungen aus. Die biospezifischen Bindungen können insbesondere durch Rezeptor-Ligand-Bindungen, wie z.B. zwischen Streptavidin und Biotin gebildet werden.

Im linken Teil von Figur 8B ist schematisch illustriert, dass die Wirkung des Haftvermittlers 4 auf einer unspezifischen Wechselwirkung 4.3 einerseits mit den thermoresponsiven Partikeln 3 und andererseits mit dem Substratkörper 1 beruhen kann.

Der Haftvermittler 4 umfasst z.B. eine Biotin-Schicht mit einer Dicke von 1 nm bis 1 µm (siehe Spinke et al. in "J. Chem. Phys." 99 (1993) 7012; Hong et al. in "Progr. Colloid Polym. Sci." 93 (1993) 98; Zao et al. in "Electroanal." 18 (2006) 1737). Die Schicht wird mit an sich bekannten Verfahren, wie z.B. Spin-Coating oder Selbstassemblierung aus der Lösung, auf der Oberfläche des Substratkörpers 1 gebildet.

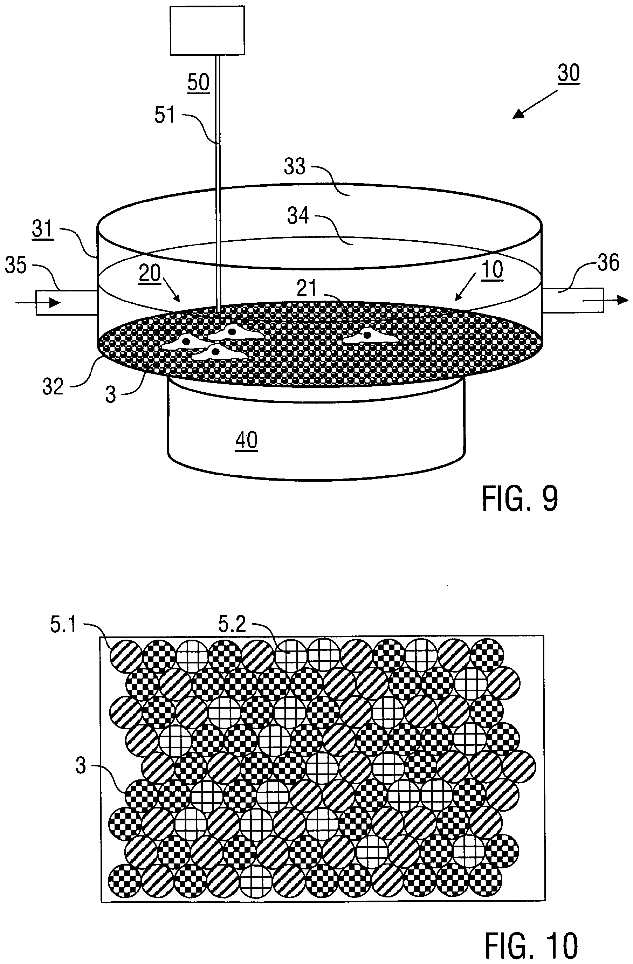

Das erfindungsgemäße Substrat 10 kann Teil einer Kultivierungseinrichtung 30 sein, wie beispielhaft in Figur 9 illustriert ist. Die Kultivierungseinrichtung 30 umfasst ein Kultivierungsgefäß 31 mit einem Boden 32 und einer umlaufenden Seitenwand 33. Das Kultivierungsgefäß 31 ist zur Aufnahme eines flüssigen Kultivierungsmediums 34 vorgesehen, das durch eine Zufuhrleitung 35 in das Kultivierungsgefäß 31 eingeführt und durch eine Auslassleitung 36 aus dem Kultivierungsgefäß 31 entfernt werden kann. Das erfindungsgemäße Substrat 10 ist auf dem Boden 32 angeordnet. Alternativ bildet der Boden 32 das Substrat 10. Auf der zum Inneren des Kultivierungsgefäßes 31 weisenden Seite des Substrats 10 sind freiliegend die thermoresponsiven Partikel 3 angeordnet. Auf dem Substrat 10 befinden sich biologische Zellen 20, 21.

Figur 9 illustriert des Weiteren schematisch eine Temperatureinstelleinrichtung 40 und eine Manipulatoreinrichtung 50. Mit der Temperatureinstelleinrichtung 40 kann die Temperatur des Substrats 10 oder von Teilbereichen (Segmenten) des Substrats 10 gezielt von einer Temperatur oberhalb bis zu einer Temperatur unterhalb der kritischen Temperatur des Phasenübergangs der thermoresponsiven Mikrogele 3 eingestellt werden. Die Temperatureinstelleinrichtung umfasst z.B. eine Heizeinrichtung, wie z. B. eine Widerstandsheizung, oder eine Kombination aus einer Heizeinrichtung und einer Kühleinrichtung, wie z. B. eine Peltier-Kühlung. Die Manipulatoreinrichtung 50 umfasst z.B. eine Zufuhrleitung 51, durch die eine Zellsuspension in das Kultivierungsgefäß 31 gespült werden kann.

Des Weiteren kann die Kultivierungseinrichtung 30 mit einer Beobachtungseinrichtung, z.B. einem Mikroskop, und einer Messeinrichtung, z.B. einem Temperatursensor (nicht dargestellt) ausgestattet sein.

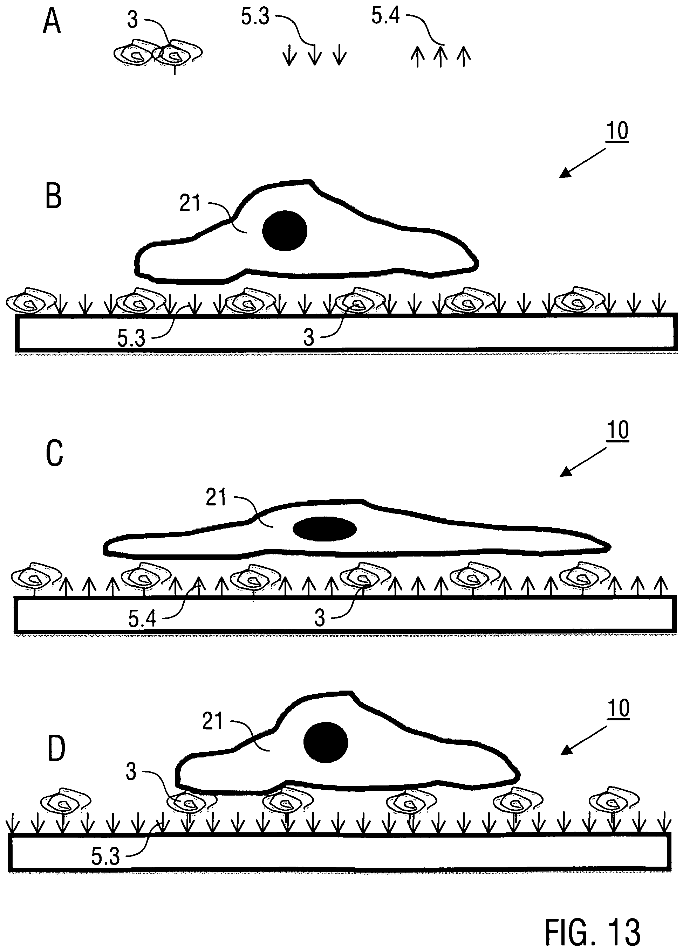

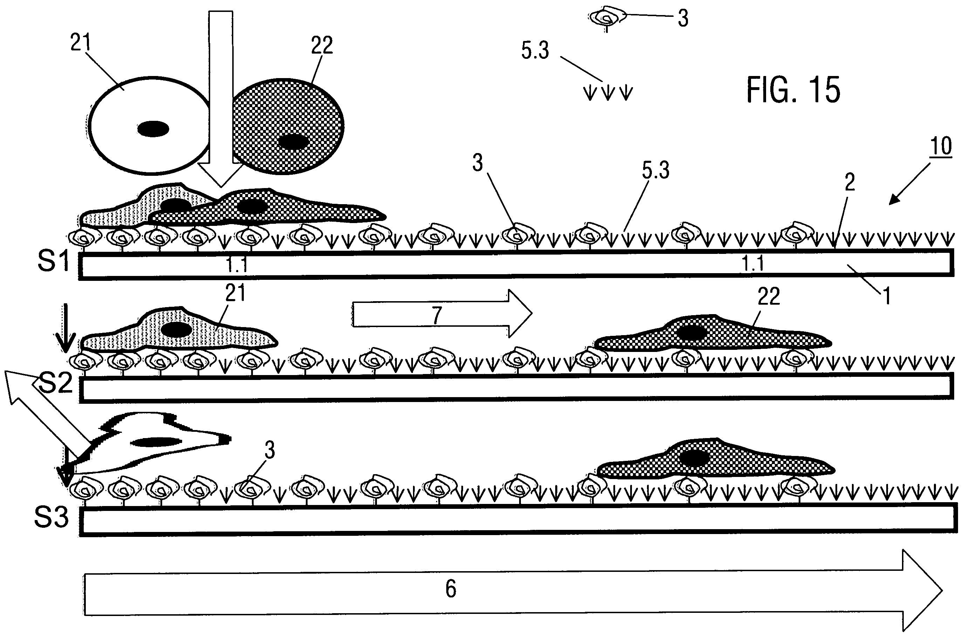

Die Figuren 10 bis 17 illustrieren verschiedene Varianten der Funktionalisierung eines Substrats mit mindestens einer Modulatorsubstanz, die erfindungsgemäß mit Modulatorpartikeln (z.B. Figuren 10 bis 12) oder nicht-erfindungsgemäß als Modulatorschicht (z.B. Figuren 13, 15) auf der Trägerfläche des Substrats vorgesehen sein kann. Allgemein umfasst die mindestens eine Modulatorsubstanz eine einzelne chemische Substanz oder eine Zusammensetzung chemischer Substanzen, zu der die biologischen Zellen eine im Vergleich zu den thermoresponsiven Mikrogelen veränderte Adhäsionsfähigkeit aufweisen (Beispiele siehe oben) und/oder mit der in den biologischen Zellen zelluläre Reaktionen induzierbar sind.

Substanzen, welche zelluläre Reaktionen auslösen, sind allgemein Substanzen, welche durch Bindung an Oberflächenrezeptoren der biologischen Zellen z.B. eine verstärkte Adhäsion, eine Migration (Zellwanderung), eine Differenzierung (insbesondere Stammzelldifferenzierung), eine Änderung des Aktivierungsstatus oder eine Änderung der Malignität bewirken. Derartige Substanzen sind z.B.:
- Chemokine, wie z.B. FGF induzieren Chemotaxis, oder
- Osteonektin (induziert Differenzierung von Stammzellen in Herzmuskelzellen).

Vorteilhafterweise ermöglicht die Kombination verschieden wirkender Modulatorsubstanzen die gezielte Einstellung vorbestimmter physikalischer oder chemischer Oberflächeneigenschaften. Da die mindestens eine Modulatorsubstanz bei der Herstellung des Mikrogels der Dispersion kolloidaler Teilchen des thermoresponsiven Polymers zugesetzt werden kann, lassen sich die thermoresponsiven Mikrogele und die mindestens eine Modulatorsubstanz wie Module frei kombinieren. Die Oberfläche des erfindungsgemäßen Substrats kann wie ein modularer Baukasten gestaltet werden.

Bei dem schematisch in Figur 10 gezeigten Beispiel sind thermoresponsive Mikrogele 3, Kunststoffpartikel, die mit zellanziehenden Molekülen beschichtet sind (adhäsionssteigernde Modulatorpartikel 5.1) und Kunststoffpartikel, die mit zellabstoßenden Molekülen beschichtet sind (adhäsionsmindernde Modulatorpartikel 5.2) kombiniert. Die Modulatorpartikel 5.1, 5.2 haben einen Durchmesser, der z.B. im Bereich 50 nm bis 1 µm gewählt ist.

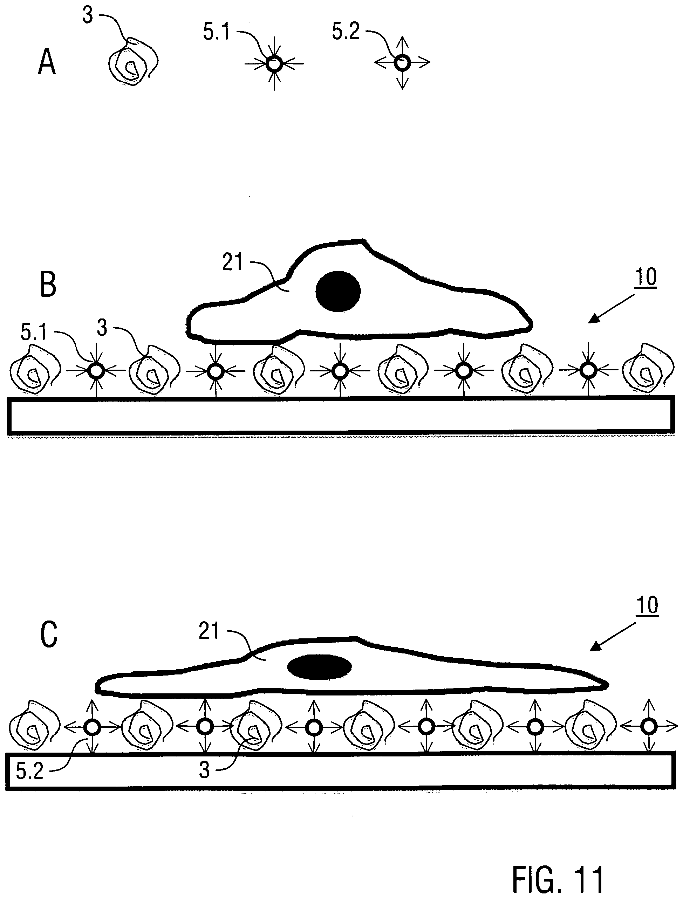

Figur 11 illustriert schematisch die verschiedenen Wirkungen der Kombination thermoresponsiver Mikrogele 3 mit adhäsionssteigernden Modulatorpartikeln 5.1 und adhäsionsmindernden Modulatorpartikeln 5.2 (symbolisiert in Figur 11A). Gemäß Figur 11B bewirken die adhäsionssteigernden Modulatorpartikel 5.1 durch eine relativ hohe Anzahl von Bindungsplätzen für die adhärente Anbindung der Zelle 21, dass eine relativ kleine Kontaktfläche zwischen der Zelle 21 und der Substratoberfläche gebildet wird. Mit der kleineren Kontaktfläche berührt die Zelle 21 relativ wenige thermoresponsive Mikrogele 3, so dass deren Wirkung bei einem temperaturabhängigen Phasenübergang vermindert wird. Im Ergebnis wird eine starke Bindung der Zelle 21 zum Substrat 10 erzielt.

Gemäß Figur 11C bewirken adhäsionsmindernde Modulatorpartikel 5.2 einen gegenteiligen Effekt. Die Zelle 21 wird auf der Oberfläche des Substrats 10 ausgebreitet, um Bindungsplätze für die Adhäsionskontakte der Zelle 21 zu finden. Entsprechend erhält die Zelle 21 Kontakt mit einer relativ großen Anzahl thermoresponsiver Mikrogele 3. Somit wirkt sich ein Phasenübergang der thermoresponsiven Mikrogele 3 stärker als bei den adhäsionssteigernden Modulatorpartikeln 5.1 (Figur 11B) aus. Die Adhäsion der Zelle 21 auf der Oberfläche des Substrats 10 wird vermindert.

Durch eine Einstellung der quantitativen Mischungsverhältnisse der thermoresponsiven Mikrogele 3 mit mindestens einem Typ der Modulatorpartikel 5.1, 5.2 in der Dispersion zur Herstellung der Mikrogele können somit vorteilhafterweise die Adhäsionseigenschaften (Anhaft- oder Ablösungsparameter) der Substratoberfläche über einen weiten Bereich variiert werden, wobei der thermoresponsive Charakter der Oberfläche erhalten bleibt und die Oberfläche für einen Zelltyp oder mehrere Zelltypen optimale Adhäsionseigenschaften besitzt. Vorteilhafterweise können die Mischungsverhältnisse in Trägerlösungen zur Herstellung der Dispersionen einfach durch Einwiegen erzeugt werden. Die Verwendung der Mikrogel-Dispersion, welche die Teilchen des thermoresponsiven Polymers und die Modulatorpartikel enthält, ermöglicht die gemeinsame Übertragung auf die Trägerfläche des Substrats in einem einzigen Depositionsschritt.

Die in der schematischen Illustration von Figur 11 gewählten Größenverhältnisse einerseits der biologischen Zelle 21 und andererseits der Mikrogele 3, 5.1 und 5.2 sind aus zeichnungstechnischen Gründen gewählt. Im Unterschied zur Illustration können erheblich geringere Partikelgrößen, z.B. bis 50 nm oder darunter, oder auch größere Partikel, z.B. 10 µm verwendet werden.

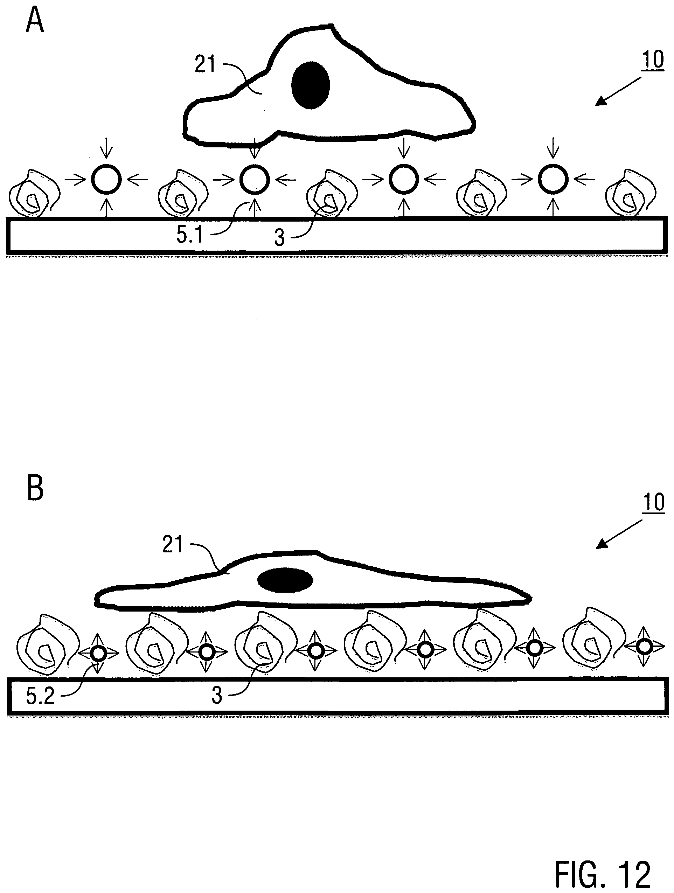

Gemäß einer weiteren Variante der Erfindung können auf der Oberfläche des Substrats 10 Partikel mit verschiedenen Größen kombiniert werden, wie beispielhaft in Figur 12 illustriert ist. Beispielsweise können die adhäsionssteigernden Modulatorpartikel 5.1 einen größeren Radius als die thermoresponsiven Mikrogele 3 haben (Figur 12A). In diesem Fall wird die adhäsionssteigernde Wirkung der Modulatorpartikel 5.1 verstärkt, da diese im Vergleich zu den thermoresponsiven Mikrogelen 3 für die Zelle 21 stärker zugänglich sind. Dazu im Gegensatz wird gemäß Figur 12B mit adhäsionsmindernden Modulatorpartikeln 5.2, deren Radius geringer als der Radius der thermoresponsiven Mikrogele 3 ist, die Wirkung der Modulatorpartikel 5.2 vermindert. Weitere Kombinationen, wie z.B. kleinere adhäsionssteigernde Modulatorpartikel 5.1 und/oder größere adhäsionsmindernde Modulatorpartikel 5.2 sind ebenfalls möglich.

Im Ergebnis können nicht nur die Adhäsionseigenschaften der Oberfläche eingestellt, sondern auch eine bestimmte Körnigkeit der Oberfläche bereitgestellt werden. Die Bereitstellung einer körnigen Oberfläche bedeutet, dass eine Oberflächentopologie mit Erhöhungen und Vertiefungen erzeugt wird. Vorteilhafterweise kann die Körnigkeit der Oberfläche an typische Dimensionen des Adhäsionsmusters eines bestimmten Zelltyps angepasst werden (Humane und Bovin Kapillarendothel-Zellen , siehe C.S. Chen et al. in "Science" 276 (1997) 1425; und C2C12-Muskelzellen, siehe U. Joos et al. in "Eur. J. Cell Bio." 85 (2006) 225).

Figur 13 illustriert eine Variante, die nicht Gegenstand der Erfindung ist, bei der die mindestens eine Modulatorsubstanz in Kombination mit den thermoresponsiven Mikrogelen 3 als adhäsionssteigernde Modulatorschicht 5.3 oder als adhäsionsmindernde Modulatorschicht 5.4 bereitgestellt wird.

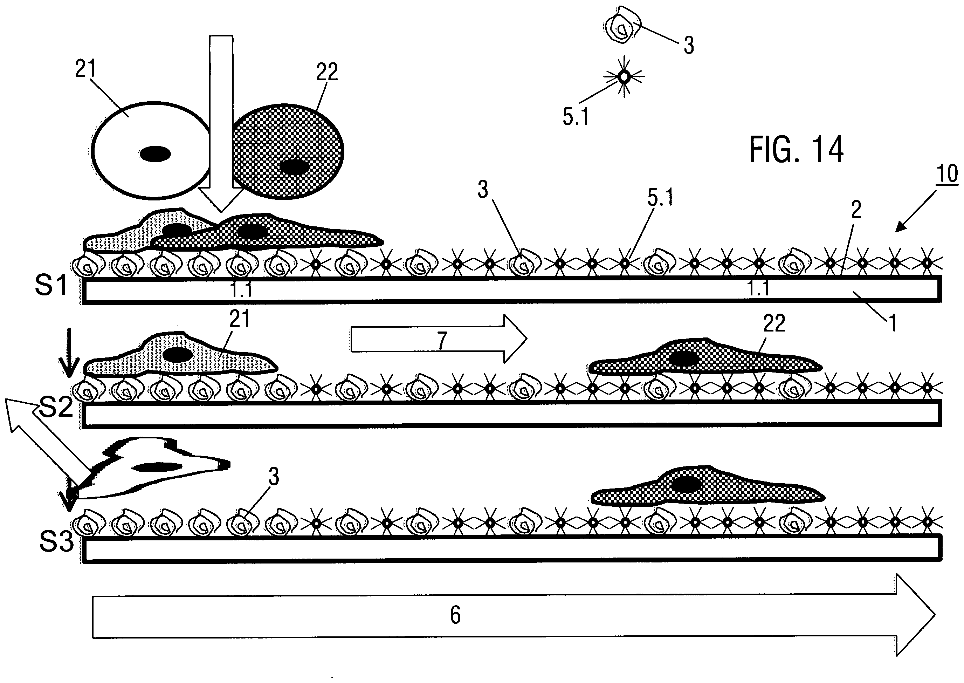

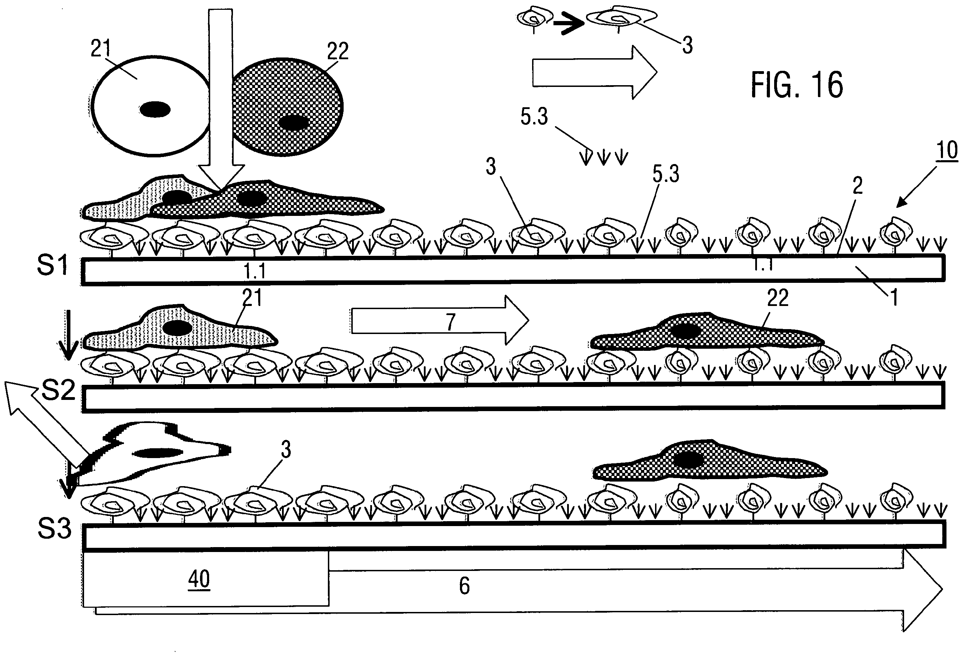

Die Figuren 14, 15 und 16 illustrieren Anordnungen, bei denen die thermoresponsiven Mikrogele, der Haftvermittler und/oder die Modulatorsubstanz auf der Trägerfläche des Substratkörpers mit mindestens einem Dichtegradienten angeordnet sind. Diese Ausführungsformen sind insbesondere für die Manipulation biologischer Zellen bei der Kultivierung in Kokulturen von Vorteil. Durch die Bildung von Dichtegradienten lassen sich die Oberflächeneigenschaften des Substrats so modifizieren, dass eine Migration (Zellwanderung) der adhärenten Zellen in Abhängigkeit vom Zelltyp induziert wird. Hierzu kann ein Konzentrationsgradient (Figuren 14, 15) und/oder ein Funktionsgradient (Figur 16) von mindestens einer Modulatorsubstanz mit chemotaktischen Eigenschaften vorgesehen sein.

Gemäß Figur 14 sind auf der Trägerfläche 2 des Substratkörpers 1 thermoresponsive Mikrogele 3 und adhäsionssteigernde Modulatorpartikel 5.1 so angeordnet, dass in einem ersten Teilbereich 1.1 eine höhere Flächendichte der thermoresponsiven Partikel 3 im Vergleich zu den adhäsionssteigernden Modulatorpartikeln 5.1 und in einem zweiten Teilbereich 1.2 umgekehrt eine geringere Dichte der thermoresponsiven Mikrogele 3 im Vergleich zu den adhäsionssteigernden Modulatorpartikeln 5.1 erzeugt wird. Im Ergebnis wird ein Dichtegradient 6 gebildet, der sich entlang der Trägerfläche 2 durch eine zunehmende Flächendichte der adhäsionssteigernden Modulatorpartikel 5.1 oder eine abnehmende Flächendichte der thermoresponsiven Mikrogele 3 auszeichnet. Der Dichtegradient 6, der in Figur 14 schematisch illustriert ist, kann in der Praxis stufenweise durch die Deposition von Mikrogelen mit verschiedenen Zusammensetzungen auf verschiedenen Teilbereichen des Substratkörpers 1 erzeugt werden.

Gemäß Figur 14 ist zur Kultivierung biologischer Zellen 21 von einem ersten, interessierenden Zelltyp gemeinsam mit Feeder-Zellen 22 die Bildung der Kokultur in dem ersten Teilbereich 1.1 mit einem hohen Anteil der thermoresponsiven Partikel 3 vorgesehen (Schritt S1). Nach der Kultivierung, die z.B. eine Differenzierung der Zellen 21 umfasst, wandern die Feeder-Zellen 22 unter der spezifischen Wirkung der adhäsionssteigernden Modulatorpartikel 5.1 aus dem ersten Teilbereich 1.1 heraus (Migration 7, Schritt S2). Anschließend erfolgt die Ablösung der interessierenden Zellen 21, indem durch eine Temperaturänderung des Substrats 10 der Phasenübergang der thermoresponsiven Mikrogele 3 induziert und im Teilbereich 1.1 die Adhäsionsfähigkeit für biologische Zellen 21 erheblich vermindert wird. Die Zellen 21 können dann, z.B. mit einer Manipulationseinrichtung, wie sie in Figur 9 gezeigt ist, vom Substrat 10 entfernt werden.

Um Zellen zelltypspezifisch zur Migration anzuregen, stehen eine Reihe von Modulatorsubstanzen zur Verfügung. Beispielsweise wirkt fMLP (Formyl Methionyl-Leucyl-Prolin) ausschließlich auf die Migration von HL 60 Leukämie-Zellen, während andere Zelltypen unbeeinflusst bleiben.

Das in Figur 14 schematisch gezeigte Prinzip der selektiven Migration 7 eines Zelltyps aus einer Mischung von adhärenten Zellen lässt sich entsprechend auf die Mischung von mehr als zwei Zelltypen verallgemeinern, wobei die adhäsionssteigernde Modulatorsubstanz so gewählt ist, dass mindestens ein Zelltyp aus der Mischung auswandert oder mindestens ein Zelltyp unverändert bleibt und keine Migration zeigt. Vorteilhafterweise können damit kleine Zellproben mit geringen Zellzahlen insbesondere unterhalb von 10⁵ Zellen nach der Kokultivierung getrennt werden.

Für die Ablösung der Zellen 21 stehen verschiedene Optionen zur Verfügung. Gemäß den Figuren 15 und 16 kann die Temperatur des gesamten Substrats 10 unter die kritische Temperatur (LCST) abgesenkt werden. Dies ist insbesondere dann möglich, wenn die adhärent bleibenden Zellen 22 im Teilbereich 1.2 vom Phasenübergang der thermoresponsiven Partikel 3 unbeeinflusst bleiben. Alternativ kann eine lokale Verminderung der Temperatur vorgesehen sein, wie schematisch in Figur 16 illustriert ist. Bei dieser Ausführungsform der Erfindung ist die Temperatureinstelleinrichtung 40 (siehe auch Figur 9) lokal auf den Teilbereich 1.1 des Substratkörpers 1 wirkend angeordnet. In diesem Fall kann der Phasenübergang der thermoresponsiven Mikrogele 3 lokal beschränkt im Teilbereich 1.1 induziert werden, während die thermoresponsiven Mikrogele in anderen Teilbereichen unverändert bleiben.

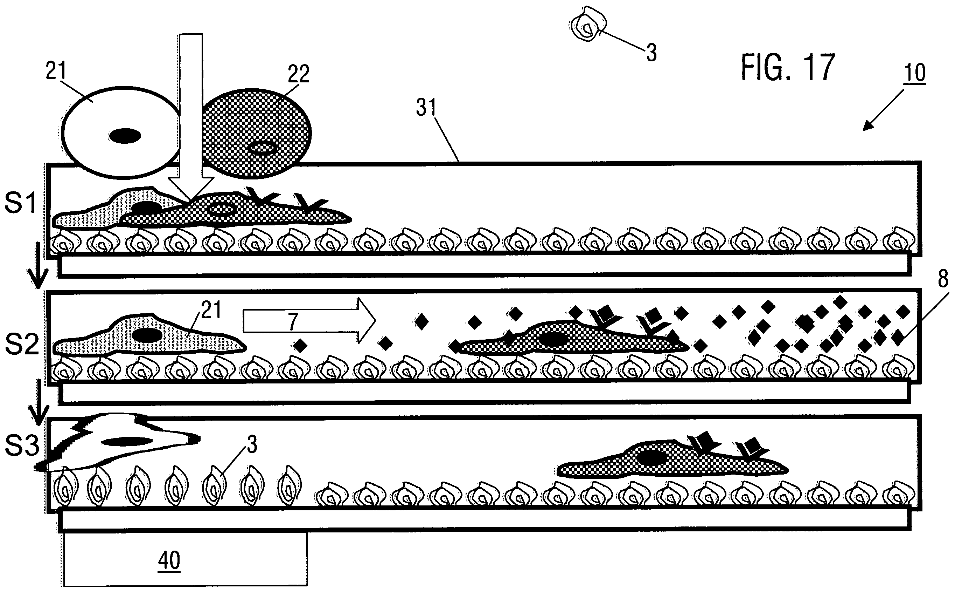

Die zelltypspezifische Migration auf der Substratoberfläche erfordert jedoch nicht zwingend einen Dichtegradienten. Alternativ oder zusätzlich können chemotaktisch wirkende Substanzen 8 dem Kultivierungsmedium zugesetzt werden, wie schematisch in Figur 17 illustriert ist.

Gemäß Figur 17 wird ein erfindungsgemäßes Substrat 10 verwendet, auf dessen Substratkörper 1 die thermoresponsiven Partikel 3 homogen verteilt angeordnet sind. Nach der Kokultivierung der Zellen 21, 22 auf dem Substrat 10 (Schritt S1) erfolgt der Zusatz chemotaktisch wirkender Substanzen 8 in das Kultivierungsmedium, so dass eine Migration 7 der Zellen induziert wird. Durch Auswahl der chemotaktisch wirkenden Substanz 8 kann die Migration 7 zelltypspezifisch induziert werden. Beispielsweise bewirkt fMLP ausschließlich eine Migration von HL 60 Leukämie-Zellen, während Zellen von Zelllinien, die aus gesundem Gewebe gewonnen wurden, unbeeinflusst bleiben.

Zur Ablösung der interessierenden Zellen 21 erfolgt bei Schritt S3 eine lokal begrenzte Temperaturabsenkung. Die thermoresponsiven Partikel 3 zeigen den Phasenübergang in dem nicht-kollabierten Zustand, so dass die Zelle 21 abgelöst werden kann.

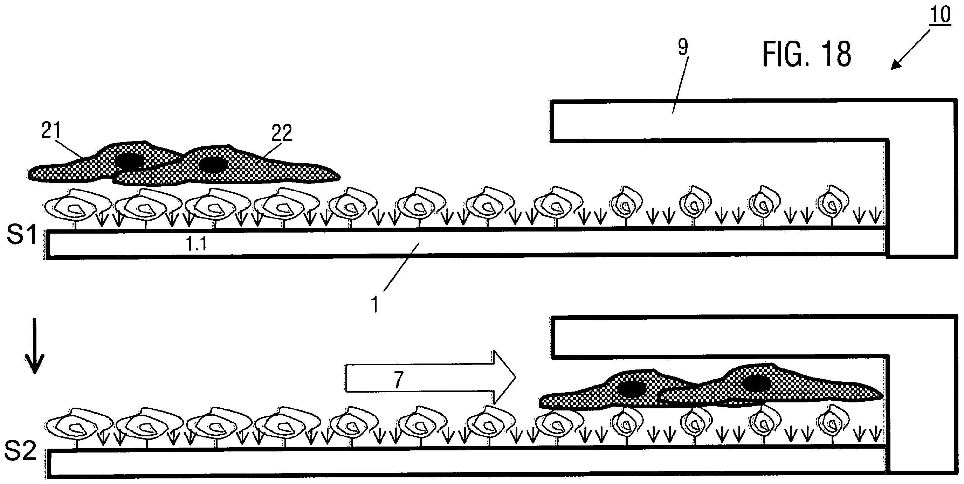

Figur 18 zeigt eine weitere Variante der Erfindung, bei der das Substrat 10 mit einer Kultivierungskavität 9 ausgestattet ist. Die Kultivierungskavität 9 kann zur *in-vitro*-Simulation von Differenzierungsvorgängen in Stammzellnischen verwendet werden. So werden im biologischen Organismus Stammzellen in Kavitäten mit einer vorbestimmten biochemischen und/oder zellulären Auskleidung vorgehalten (siehe David T. Scadden in "Nature" 441 (2006) 1075; M.C. Dusseiller et al. in "Biointerphases" 1 (2006) P1) und einer Differenzierung unterzogen.

Ein Beispiel einer derartigen Stammzellnische sind Haarfollikeln.

Mit der Kultivierungskavität 9 des erfindungsgemäßen Substrats 10 wird eine Mikroumgebung für biologische Zellen 21 geschaffen, in der die Bedingungen im Organismus nachgebildet werden. Herkömmliche Zellmanipulationstechniken zur Auskleidung künstlicher Kultivierungskavitäten mit biologischen Zellen erfordern die Verwendung optischer Pinzetten oder dielektrophoretisch wirkender Elemente. Dies ist hinsichtlich des Geräteaufwands und der komplexen Verfahren nachteilig. Dieses Problem wird mit dem erfindungsgemäßen Substrat gemäß Figur 18 dadurch gelöst, dass Zellen gezielt in die Kultivierungskavität 9 einwandern.

Gemäß Schritt S1 in Figur 18 wird ein Zellgemisch aus biologischen Zellen 21, 22, die in der Kultivierungskavität 9 angeordnet werden sollen, in einem ersten Teilbereich 1.1 des Substratkörpers 1 aufgebracht und ggf. kultiviert. Bei einem zweiten Schritt S2 erfolgt die gezielte Migration der Zellen 21, 22 in die Kultivierungskavität 9, wobei einer der oben genannten Mechanismen, z.B. ein Dichtegradient und/oder eine aus dem Kulturmedium wirkende chemotaktische Substanz verwendet werden.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Substrat (10), insbesondere zur Aufnahme biologischer Zellen (20, 21, 22), umfassend
- einen Substratkörper (1), der eine Trägerfläche (2) aufweist,
**dadurch gekennzeichnet, dass**
- auf der Trägerfläche (2) thermoresponsive Mikrogele (3), d.h. Partikel, die ein thermoresponsives Polymer enthalten, mit einem Durchmesser von 10 nm bis 10 µm fixiert sind und ferner bei dem Substrat mindestens eine Modulatorsubstanz (5) vorgesehen ist, mit der biologische Zellen eine Adhäsionsfähigkeit aufweisen, die sich von der Adhäsionsfähigkeit der biologischen Zellen an den thermoresponsiven Mikrogelen (3) unterscheidet, und/oder mit der durch Bindung an Oberflächenrezeptoren der biologischen Zellen zelluläre Reaktionen induzierbar sind, und
wobei die mindestens eine Modulatorsubstanz (5) auf Modulatorpartikeln (5.1, 5.2) angeordnet ist.

2. Substrat gemäß Anspruch 1, bei dem
- die thermoresponsiven Mikrogele (3) aus mindestens einem ungeladenen und nichtionisierbaren Polymer gebildet sind.

3. Substrat gemäß einem der vorhergehenden Ansprüche, bei dem die thermoresponsiven Mikrogele (3) aus mindestens einem der Polymere (Homo- oder Copolymere) gebildet sind: 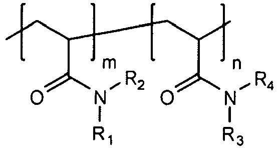
mit R₁, R₂, R₃ und R₄ = H oder Alkyl, bevorzugt R₁ = Isopropyl, R₂ = H, n = 0, 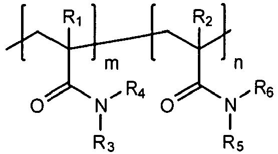
mit R₁, R₂ = -H, -Alkyl, bevorzugt -H und -CH₃, besonders bevorzugt -H,
mit R₃, R₉, R₅ und R₆ = -H, -Alkyl, Alkenyl, Alkinyl, Aryl, bevorzugt -R₃ = -Isopropyl, -R₄ = -H, -R₅ und -R₆ = -C₂H₅, -CH₃ oder -H, besonders bevorzugt -R₃ = -Isopropyl und -R₄ = -H und
m : n = 100 : 0 und / oder R₃, R₄, R₅ und 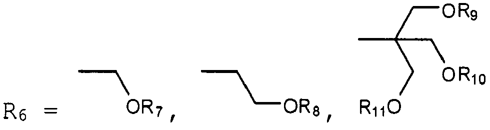
mit R₇, bis R₁₁ = -H, -Alkyl, Alkenyl, Alkinyl, Aryl, -Alkyloyl, mindestens ein R = H, 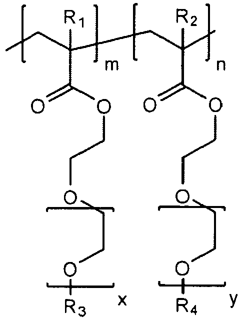
mit R₁, R₂ = H oder CH₃, R₃, R₄ = H oder Alkyl, x, y = 0 bis 20, 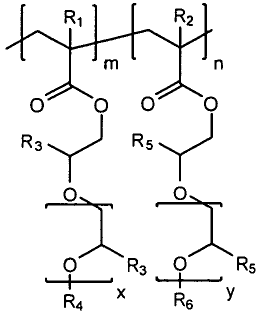
mit R₁, R₂ = -H, -Alkyl, bevorzugt -H und -CH₃, besonders bevorzugt -CH₃
mit R₃, R₅ = -H, -Alkyl, Alkenyl, Alkinyl, Aryl, bevorzugt -H und -CH₃, besonders bevorzugt -H, in jedem Fall muß gelten: wenn R₃, ≠ -H, dann R₅ = -H,
mit R₄, R₆ = -H, -Alkyl, Alkenyl, Alkinyl, Aryl, bevorzugt -H und / oder -CH₃, besonders bevorzugt -CH₃, wobei
wenn R₃, R₅ = -H und R₄, R₆ = -CH₃, x = 1 und y = 7,5, dann liegt m : n bevorzugt zwischen 95 : 5 und 90 : 10, besonders bevorzugt bei 93 : 7, und
wenn R₃, R₅ = -H und R₄, R₆ = -CH₃, x = 1 und y = 4,5, dann liegt m : n bevorzugt zwischen 93 : 7 und 80 : 20, und besonders bevorzugt bei 85 : 15, 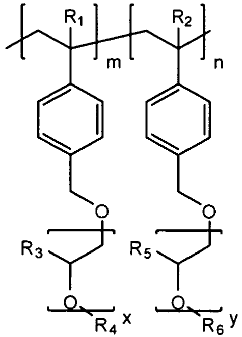
mit R₁, R₂ = -H, -Alkyl, bevorzugt -H und -CH₃, besonders bevorzugt -H,
mit R₃, R₅ = -H, -Alkyl, Alkenyl, Alkinyl, Aryl, bevorzugt -H und -CH₃, besonders bevorzugt -H, in jedem Fall muß gelten: wenn R₃, ≠ -H, dann R₅ = -H,
mit R₄, R₆ = -H, -Alkyl, Alkenyl, Alkinyl, Aryl, bevorzugt -H und / oder -CH₃, besonders bevorzugt -H, wobei
wenn R₁, R₂ = -H, und R₄, R₆ = -H, x =3 und y = 4, dann liegt m : n bevorzugt zwischen 65 : 35 und 45 : 55, besonders bevorzugt zwischen 60 : 40 und 50 : 50, 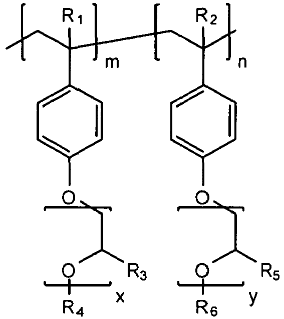
mit R₁, R₂ = -H, -Alkyl, bevorzugt -H und -CH₃, besonders bevorzugt -H,
mit R₃, R₅ = -H, -Alkyl, Alkenyl, Alkinyl, Aryl, bevorzugt -H und -CH₃, besonders bevorzugt -H, wobei
wenn R₃, ≠ -H, dann R₅ = -H
mit R₄, R₆ = -H, -Alkyl, Alkenyl, Alkinyl, Aryl, bevorzugt -H und / oder -CH₃, besonders bevorzugt -H, 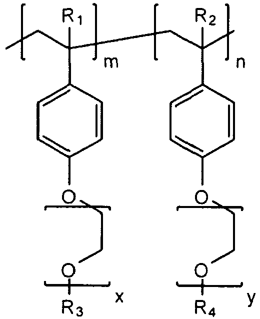
mit R₁, R₂ = H oder CH₃, R₃, R₄ = H oder Alkyl, x, y = 2 bis 20, 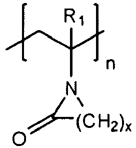
mit R₁ = H oder CH₃, x = 3 bis 5, Copolymere mit x = 3 und X > 3, 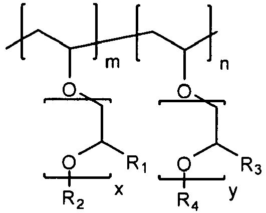
mit R₁, R₃ = -H, -Alkyl, Alkenyl, Alkinyl, Aryl, bevorzugt -H und -CH₃, besonders bevorzugt -H, in jedem Fall muß gelten:
wenn R₃, ≠ -H, dann R₅ = -H,
mit R₂, R₄ = -H, -Alkyl, Alkenyl, Alkinyl, Aryl, bevorzugt -H und / oder -CH₃, besonders bevorzugt -CH₃, 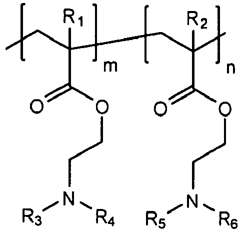 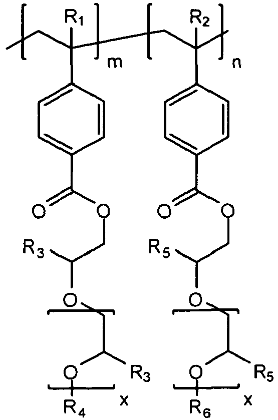
mit R₁, R₂ = -H, -Alkyl, bevorzugt -H und -CH₃, besonders bevorzugt -H,
mit R₃, R₅ = -H, -Alkyl, Alkenyl, Alkinyl, Aryl, bevorzugt -H und -CH₃, besonders bevorzugt -H, wobei:
wenn R₃, ≠ -H, dann R₅ = -H,
mit R₄, R₆ = -H, -Alkyl, Alkenyl, Alkinyl, Aryl, bevorzugt -H und / oder -CH₃, besonders bevorzugt -H, 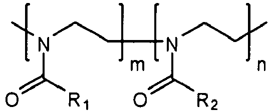
mit R₁, R₂ = -H, -Alkyl, Alkenyl, Alkinyl, Aryl, 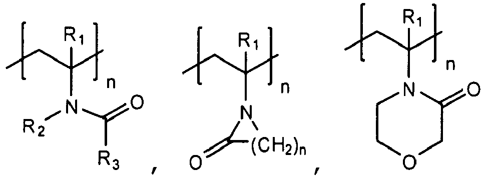 oder Copolymere der drei Elemente in Zusammensetzungen,
mit R₁ = -H, -Alkyl, bevorzugt -H und -CH₃, besonders bevorzugt -H,
mit R₂, R₃ = -H, -Alkyl, Alkenyl, Alkinyl, Aryl mit 2 ≤ n ≤ 10, bevorzugt 3 ≤ n ≤ 6, 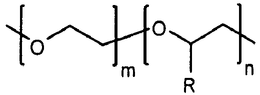
mit -R = -H, -Alkyl, Alkenyl, Alkinyl, Aryl, bevorzugt -R = - Alkyl, besonders bevorzugt -R = -CH₃, 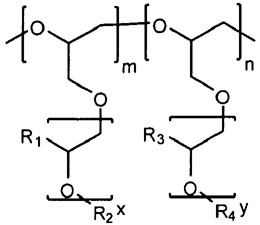
mit R₁, R₃ = -H, -Alkyl, Alkenyl, Alkinyl, Aryl, bevorzugt -H und -CH₃, wobei
wenn R₁, ≤ -H, dann R₃ = -H,
mit R₂, R₄ = -H, -Alkyl, Alkenyl, Alkinyl, Aryl, bevorzugt -H und / oder -CH₃, 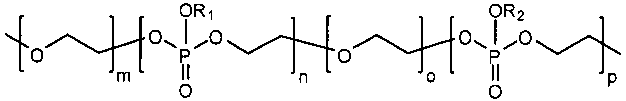
mit R₁, R₂ = -H, -Alkyl, Alkenyl, Alkinyl, Aryl, 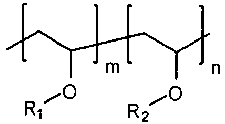
mit R₁, R₂ = -H, -Alkyl, Alkenyl, Alkinyl, Aryl, bevorzugt R₁ = -CH₃, n = 0,
und analoge Strukturen, basierend auf anderen Polysaccharidgerüsten als Cellulose, 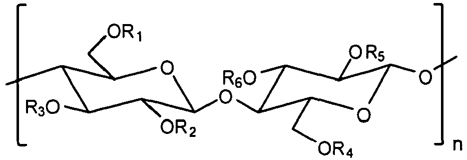
mit R₁ bis R₆ = -H, -Alkyl, Alkenyl, Alkinyl, Aryl, -Alkyloyl, mindestens zwei R = H,
Homo- oder Copolymere mit elastin-ähnlichen Einheiten, und
Copolymere aller oben genannten Einheiten mit veränderten Monomeren.

4. Substrat gemäß Anspruch 3, bei dem mindestens eine terminale Einheit der Polymerhauptkette eine Kopplungsgruppe zur Trägerfläche (2) beinhaltet.

5. Substrat gemäß einem der vorhergehenden Ansprüche, bei dem die thermoresponsiven Mikrogele (3) aus mindestens einem der Polymere gebildet sind: - Poly-(N-isopropylacrylamid),
- -X-(-CH₂-CR₁COO-R₂-)ₙ-(-CH₂-CR₁COO-R₃-)ₘ-R₄ oder Copolymere davon, und
- -X-[(-CH₂-CR₁COO-R₂-)ₙ-(-CH₂-CR₁COO-R₃-)ₘ-R₄]₂ oder Copolymere davon, wobei
X eine Kopplungsgruppe zur Trägerfläche, R₁ = H oder CH₃, R₂/R₃ = aliphatische Kohlenwasserstoffketten mit Ethergruppen, und R₄ H, eine aliphatische Kohlenwasserstoffkette oder eine funktionelle Gruppe ist.

6. Substrat gemäß einem der vorhergehenden Ansprüche, bei dem
- die thermoresponsiven Mikrogele (3) aus mindestens zwei verschiedenen Polymeren gebildet sind und/oder verschiedene Durchmesser aufweisen.

7. Substrat gemäß einem der vorhergehenden Ansprüche, bei dem
- die thermoresponsiven Mikrogele (3) einen Durchmesser aufweisen, der mindestens 50 nm und/oder höchstens 10 µm beträgt.

8. Substrat gemäß einem der vorhergehenden Ansprüche, bei dem
- die thermoresponsiven Mikrogele (3) eine Kern-Schale-Struktur aufweisen.

9. Substrat gemäß Anspruch 8, mit mindestens einem der Merkmale
- ausschließlich die Schale der thermoresponsiven Mikrogele (3) ist thermoresponsiv,
- der Zusammenhalt der Kerne der thermoresponsiven Mikrogele (3) wird durch Nebenvalenzwechselwirkungen bewirkt,
- der Zusammenhalt der Kerne der thermoresponsiven Mikrogele (3) wird durch chemische Vernetzung bewirkt,
- Polymerketten in der Schale der thermoresponsiven Mikrogele (3) sind unvernetzt,
- Polymerketten in der Schale der thermoresponsiven Mikrogele (3) sind vernetzt, wobei die Anzahl von Vernetzungspunkte nicht größer ist als 1 pro 20 unvernetzte Wiederholungseinheiten ist, und
- die Dicke der Schale der thermoresponsiven Mikrogele (3) beträgt mindestens 10 nm.

10. Substrat gemäß einem der vorhergehenden Ansprüche, bei dem
- die thermoresponsiven Mikrogele (3) zusammen mit den Modulatorpartikeln (5.1, 5.2) eine Monolage, insbesondere eine geschlossene Monolage, bilden.

11. Substrat gemäß einem der vorhergehenden Ansprüche, bei dem
- die Trägerfläche (2) mit einem Haftvermittler (4) versehen ist.

12. Substrat gemäß einem der vorhergehenden Ansprüche, bei dem
- mindestens eines von den thermoresponsiven Mikrogelen (3), dem Haftvermittler und der Modulatorsubstanz auf der Trägerfläche (2) mindestens einen Dichtegradienten (6) bilden.

13. Substrat gemäß einem der vorhergehenden Ansprüche, bei dem
- auf der Trägerfläche (2) mindestens eine Kultivierungskavität (9) vorgesehen ist.

14. Substrat gemäß einem der vorhergehenden Ansprüche, bei dem
- der Substratkörper (1) Teil einer Kultivierungseinrichtung (30) ist.

15. Verfahren zur Herstellung eines Substrates (10) gemäß einem der vorhergehenden Ansprüche, mit den Schritten:
- Bereitstellung des Substratkörpers (1) mit der Trägerfläche (2),
- Herstellung einer Dispersion der thermoresponsiven Mikrogele (3),
- Auftragung der Dispersion auf der Trägerfläche (2), und
- Fixierung der thermoresponsiven Mikrogele (3) auf der Trägerfläche (2),
wobei das Verfahren ferner die Auftragung von mindestens einer Modulatorsubstanz (5) auf Modulatorpartikeln auf der Trägerfläche (2) umfasst.

16. Verfahren gemäß Anspruch 15, mit mindestens einem der Schritte
- Auftragung eines Haftvermittlers (4) auf der Trägerfläche, und
- Sterilisierung der Trägerfläche (2).

17. Verfahren zur Kultivierung biologischer Zellen (20, 21, 22) auf einem Substrat (10) gemäß einem der Ansprüche 1 bis 14, mit den Schritten:
- Ablage der biologischer Zellen (20, 21, 22) auf dem Substrat (10), und
- Einstellung von Kultivierungsbedingungen derart, dass die biologischen Zellen (20) einem Wachstum, einer Differenzierung und/oder einer Migration (7) unterzogen werden.

18. Verfahren gemäß Anspruch 17, mit mindestens einem der Schritte
- Einstellung der Adhäsion der biologischen Zellen (20, 21, 22) auf dem Substrat (10) durch eine Temperatureinstellung,
- Einstellung einer zelltypspezifischen Migration (7) von mindestens einem Typ der biologischer Zellen (20, 21, 22) mittels eines Dichtegradienten einer zelltypspezifisch wirkenden Modulatorsubstanz, und
- Einstellung der Migration (7) von mindestens einem Typ der biologischen Zellen (20, 21, 22) mittels eines Dichtegradienten einer Modulatorsubstanz derart, dass die biologischen Zellen (20, 21, 22) in eine Kultivierungskavität (9) wandern.

## Claims

1. A substrate (10), in particular for receiving biological cells (20, 21, 22), comprising
- a substrate body (1) having a carrier area (2), **characterized in that**
- thermoreactive microgels (3), i.e. particles which comprise a thermoreactive polymer, having a diameter of 10 nm to 10 µm are fixed on the carrier area (2) and further the substrate is provided with at least one modulator substance (5) with which biological cells have an adhesion capability which differs from the adhesion capability of the biological cells to the thermoreactive microgels (3), and/or with which cellular reactions are inducible by binding to surface receptors of the biological cells, and
wherein the at least one modulator substance (5) is disposed on modulator particles (5.1, 5.2).

2. The substrate according to claim 1, in which
- the thermoreactive microgels (3) are formed from at least one uncharged and non-ionizable polymer.

3. The substrate according to any one of the preceding claims, in which the thermoreactive microgels (3) are formed from at least one of the polymers (homo- or copolymers) : 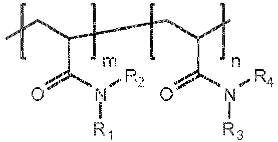
with R₁, R₂, R₃ and R₄ = H or alkyl, preferably R₁ = isopropyl, R₂ = H, n = 0, 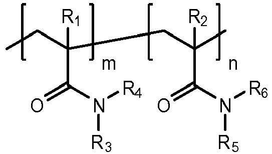
with R₁, R₂ = -H, -alkyl, preferably -H and -CH₃, particularly preferably -H,
with R₃, R₄, R₅ and R₆ = -H, -alkyl, alkenyl, alkynyl, aryl, preferably -R₃ = -isopropyl, -R₄ = -H, -R₅ and -R₆ = -C₂H₅, -CH₃ or -H, particularly preferably -R₃ = -isopropyl and -R₄ = -H and m : n = 100 : 0 and/or R₃, R₄, R₅ and R₆ = 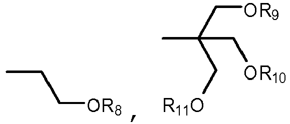
with R₇ to R₁₁ = -H, -alkyl, alkenyl, alkynyl, aryl, -alkyloyl, at least one R = H, 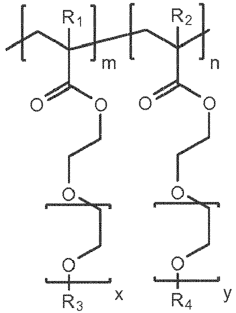
with R₁, R₂ = H or CH₃, R₃, R₄ = H or alkyl, x, y = 0 to 20, 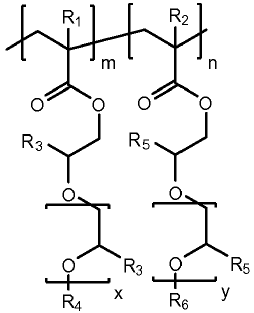
with R₁, R₂ = -H, -alkyl, preferably -H and -CH₃, particularly preferably -CH₃
with R₃, R₅ = -H, -alkyl, alkenyl, alkynyl, aryl, preferably -H and -CH₃, particularly preferably -H, the following having to be applied in any case: if R₃ ≠ -H, R₅ = -H,
with R₄, R₆ = -H, -alkyl, alkenyl, alkynyl, aryl, preferably -H and/or -CH₃, particularly preferably -CH₃, wherein
if R₃, R₅ = -H and R₄, R₆ = -CH₃, x = 1 and y = 7.5, m : n is preferably between 95 : 5 and 90 : 10, particularly preferably at 93 : 7, and
if R₃, R₅ = -H and R₄, R₆ = -CH₃, x = 1 and y = 4.5, m : n is preferably between 93 : 7 and 80 : 20 and particularly preferably at 85 : 15, 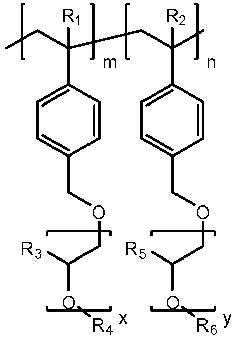
with R₁, R₂ = -H, -alkyl, preferably -H and -CH₃, particularly preferably -H,
with R₃, R₅ = -H, -alkyl, alkenyl, alkynyl, aryl, preferably -H and -CH₃, particularly preferably -H, the following having to be applied in any case: if R₃ ≠ -H, R₅ = -H,
with R₄, R₆ = -H, -alkyl, alkenyl, alkynyl, aryl, preferably -H and/or -CH₃, particularly preferably -H, wherein
if R₁, R₂ = -H and R₄, R₆ = -H, x =3 and y = 4, m : n is preferably between 65 : 35 and 45 : 55, particularly preferably between 60 : 40 and 50 : 50, 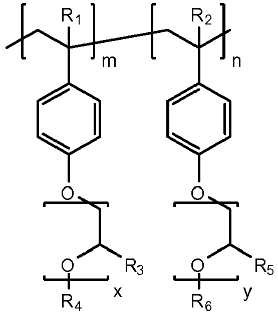
with R₁, R₂ = -H, -alkyl, preferably -H and -CH₃, particularly preferably -H,
with R₃, R₅ = -H, -alkyl, alkenyl, alkynyl, aryl, preferably -H and -CH₃, particularly preferably -H, wherein
if R₃ ≠ -H, R₅ = -H
with R₄, R₆ = -H, -alkyl, alkenyl, alkynyl, aryl, preferably -H and/or -CH₃, particularly preferably -H, 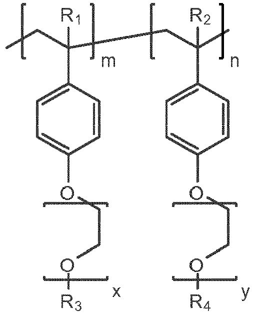
with R₁, R₂ = H or CH₃, R₃, R₄ = H or alkyl, x, y = 2 to 20, 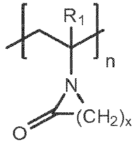
with R₁ = H or CH₃, x = 3 to 5, copolymers with x = 3 and X > 3, 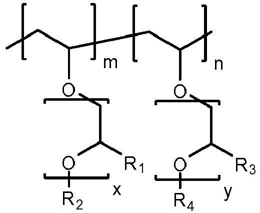
with R₁, R₃ = -H, -alkyl, alkenyl, alkynyl, aryl, preferably -H and -CH₃, particularly preferably -H, the following having to be applied in any case: if R₃ ≠ -H, R₅ = -H,
with R₂, R₄ = -H, -alkyl, alkenyl, alkynyl, aryl, preferably -H and/or -CH₃, particularly preferably -CH₃, 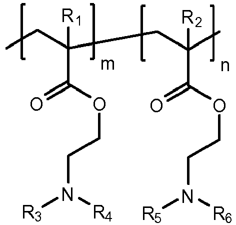 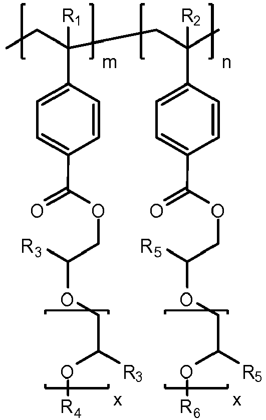
with R₁, R₂ = -H, -alkyl, preferably -H and -CH₃, particularly preferably -H,
with R₃, R₅ = -H, -alkyl, alkenyl, alkynyl, aryl, preferably -H and -CH₃, particularly preferably -H, wherein:
if R₃ ≠ -H, R₅ = -H,
with R₄, R₆ = -H, -alkyl, alkenyl, alkynyl, aryl, preferably -H and/or -CH₃, particularly preferably -H, 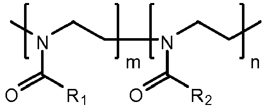
with R₁, R₂ = -H, -alkyl, alkenyl, alkynyl, aryl, 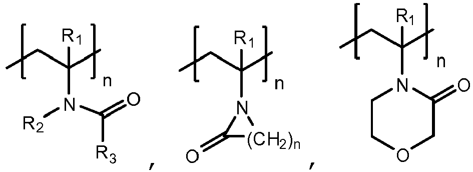 or copolymers of the three elements in compositions,
with R₁ = -H, -alkyl, preferably -H and -CH₃, particularly preferably -H,
with R₂, R₃ = -H, -alkyl, alkenyl, alkynyl, aryl
with 2 ≤ n ≤ 10, preferably 3 ≤ n ≤ 6, 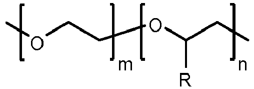
with -R = -H, -alkyl, alkenyl, alkynyl, aryl, preferably -R = -alkyl, particularly preferably -R = -CH₃, 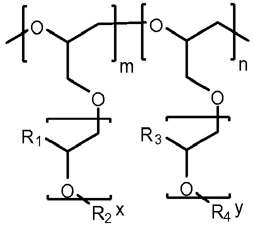
with R₁, R₃ = -H, -alkyl, alkenyl, alkynyl, aryl, preferably -H and -CH₃, wherein
if R₁ ≠ -H, R₃ = -H,
with R₂, R₄ = -H, -alkyl, alkenyl, alkynyl, aryl, preferably -H and/or -CH₃, 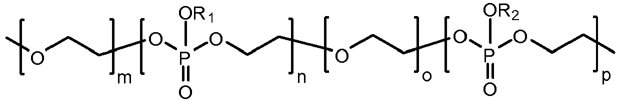
with R₁, R₂ = -H, -alkyl, alkenyl, alkynyl, aryl, 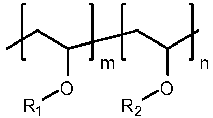
with R₁, R₂ = -H, -alkyl, alkenyl, alkynyl, aryl, preferably R₁ = -CH₃, n = 0,
and analogous structures, based on other polysaccharide scaffolds than cellulose, 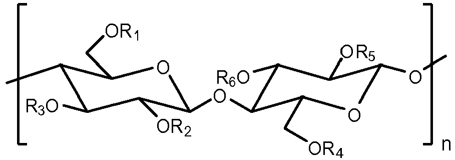
with R₁ to R₆ = -H, -alkyl, alkenyl, alkynyl, aryl, -alkyloyl, at least two R = H,
homo- or copolymers with elastine-like units, and
copolymers of all the above-mentioned units with changed monomers.

4. The substrate according to claim 3, in which at least one terminal unit of the polymer backbone includes a coupling group to the carrier area (2).

5. The substrate according to any one of the preceding claims, in which the thermoreactive microgels (3) are formed from at least one of the polymers: -poly-(N-isopropyl acrylamide),
- -X-(-CH₂-CR₁COO-R₂-)ₙ-(-CH₂-CR₁COO-R₃-)ₘ-R₄ or copolymers thereof, and
- -X- [(-CH₂-CR₁COO-R₂-)ₙ-(-CH₂-CR₁COO-R₃-)ₘ-R₄]₂ or copolymers thereof, wherein
X is a coupling group to the carrier area, R₁ = H or CH₃, R₂/R₃ = aliphatic hydrocarbon chains with ether groups, and R₄ is H, an aliphatic hydrocarbon chain or a functional group.

6. The substrate according to any one of the preceding claims, in which - the thermoreactive microgels (3) are formed from at least two different polymers and/or have different diameters.

7. The substrate according to any one of the preceding claims, in which
- the thermoreactive microgels (3) have a diameter which is at least 50 nm and/or at most 10 µm.

8. The substrate according to any one of the preceding claims, in which
- the thermoreactive microgels (3) have a core-shell structure.

9. The substrate according to claim 8, having at least one of the features
- only the shell of the thermoreactive microgels (3) is thermoreactive,
- the cohesion of the cores of the thermoreactive microgels (3) is caused by secondary-valence interactions,
- the cohesion of the cores of the thermoreactive microgels (3) is caused by chemical cross-linking,
- polymer chains in the shell of the thermoreactive microgels (3) are not cross-linked,
- polymer chains in the shell of the thermoreactive microgels (3) are cross-linked, the number of cross-linking points being no higher than 1 per 20 not cross-linked repeating units, and
- the thickness of the shell of the thermoreactive microgels (3) is at least 10 nm.

10. The substrate according to any one of the preceding claims, in which
- the thermoreactive microgels (3) together with the modulator particles (5.1, 5.2) form a monolayer, in particular a closed monolayer.

11. The substrate according to any one of the preceding claims, in which
- the carrier area (2) is provided with an adhesion promoter (4).

12. The substrate according to any one of the preceding claims, in which
- at least one of the thermoreactive microgels (3), the adhesion promoter and the modulator substance form at least one density gradient (6) on the carrier area (2).

13. The substrate according to any one of the preceding claims, in which
- at least one cultivation cavity (9) is provided on the carrier area (2).

14. The substrate according to any one of the preceding claims, in which
- the substrate body (1) is part of a cultivation device (30).

15. A method for the preparation of a substrate (10) according to any one of the preceding claims, with the steps:
- provision of a substrate body (1) having the carrier area (2),
- preparation of a dispersion of the thermoreactive microgels (3),
- application of the dispersion to the carrier area (2), and
- fixing of the thermoreactive microgels (3) on the carrier area (2),
wherein the method further comprises the application of at least one modulator substance (5) on modulator particles to the carrier area (2).

16. The method according to Claim 15, with at least one of the steps of
- application of an adhesion promoter (4) to the carrier area (2), and
- sterilization of the carrier area (2).

17. A method for the cultivation of biological cells (20, 21, 22) on a substrate (10) according to any one of Claims 1 to 14, with the steps:
- deposition of the biological cells (20, 21, 22) on the substrate (10), and
- setting of cultivation conditions such that the biological cells (20) are subjected to a growth, a differentiation and/or a migration (7).

18. The method according to Claim 17, with at least one of the steps of
- setting of the adhesion of the biological cells (20, 21, 22) on the substrate (10) by setting the temperature,
- setting a cell type-specific migration (7) of at least one type of the biological cells (20, 21, 22) via a density gradient of a cell type-specifically acting modulator substance, and
- setting of the migration (7) of at least one type of the biological cells (20, 21, 22) via a density gradient of a modulator substance such that the biological cells (20, 21, 22) migrate into a cultivation cavity (9).

## Revendications

1. Substrat (10), en particulier pour recevoir des cellules biologiques (20, 21, 22), comprenant
- un corps de substrat (1) qui présente une surface de support (2),
**caractérisé en ce que**
- sur la surface de support (2) sont fixés des microgels thermorépondeurs (3), c'est-à-dire des particules qui contiennent un polymère thermorépondeur avec un diamètre de 10 nm à 10 µm et en outre pour le substrat, au moins une substance modulatrice (5) est prévue, avec laquelle des cellules biologiques présentent une capacité d'adhérence qui se distingue de la capacité d'adhérence des cellules biologiques sur les microgels thermorépondeurs (3), et/ou avec laquelle, par liaison à des récepteurs de surface des cellules biologiques, des réactions cellulaires sont inductibles, et
dans lequel l'au moins une substance modulatrice (5) est disposée sur des particules modulatrices (5.1, 5.2).

2. Substrat selon la revendication 1, dans lequel
- les microgels thermorépondeurs (3) sont formés d'au moins un polymère non chargé et non ionisable.

3. Substrat selon l'une des revendications précédentes, dans lequel les microgels thermorépondeurs (3) sont formés d'au moins l'un des polymères (homopolymère ou copolymère) : 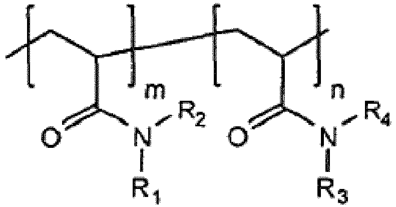
où R₁, R₂, R₃ et R₄ = H ou un groupe alkyle, de préférence R₁ = un groupe isopropyle, R₂ = H, n = 0, 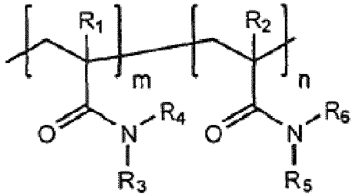
où R₁, R₂ = -H, un groupe -alkyle, de préférence - H et -CH₃, de manière particulièrement préférée, -H,
où R₃, R₄, R₅ et R₆ = -H, un groupe -alkyle, alcényle, alcinyle, aryle, de préférence, -R₃ = un groupe -isopropyle, -R₄ = -H, -R₅ et -R₆ = -C₂H₅, -CH₃ ou -H, de manière particulièrement préférée, -R₃ = un groupe -isopropyle et -R₄ = -H et m : n = 100 : 0 et/ou R₃, R₄, R₅ et R₆ = 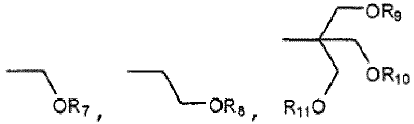
où R₇, jusqu'à R₁₁ = -H, un groupe -alkyle, alcényle, alcinyle, aryle, -alkyloyle, au moins un R = H, 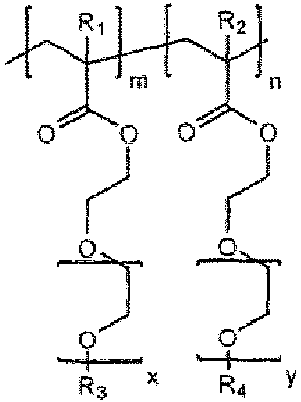
où R₁, R₂ = H ou CH₃, R₃, R₄ = H ou un groupe alkyle, x, y = 0 à 20, 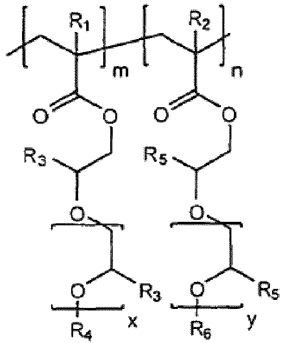
où R₁, R₂ = -H, un groupe -alkyle, de préférence - H et -CH₃, de manière particulièrement préférée, -CH₃
où R₃, R₅ = -H, un groupe -alkyle, alcényle, alcinyle, aryle, de préférence, -H et -CH₃, de manière particulièrement préférée, -H, dans chaque cas : si R₃ ≠ -H, alors R₅ = -H,
où R₄, R₆ = ≠ -H, un groupe -alkyle, alcényle, alcinyle, aryle, de préférence -H et/ou -CH₃, de manière particulièrement préférée, -CH₃, dans lequel
si R₃, R₅ = -H et R₄, R₆ = -CH₃, x = 1 et y = 7, 5, alors m : n se situe de préférence entre 95 : 5 et 90 : 10, de manière particulièrement préférée, à 93 : 7, et
si R₃, R₅ = -H et R₄, R₆ = -CH₃, x = 1 et y = 4,5, alors m : n se situe de préférence entre 93 : 7 et 80 : 20 et de manière particulièrement préférée, à 85 : 15, 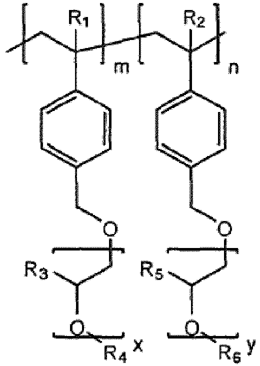
où R₁, R₂ = -H, un groupe -alkyle, de préférence - H et -CH₃, de manière particulièrement préférée, -H,
où R₃, R₅ = -H, un groupe -alkyle, alcényle, alcinyle, aryle, de préférence, -H et -CH₃, de manière particulièrement préférée, -H, dans chaque cas : si R₃ ≠ -H, alors R₅ = -H,
où R₄, R₆ = -H, un groupe -alkyle, alcényle, alcinyle, aryle, de préférence -H et/ou -CH₃, de manière particulièrement préférée, H, dans lequel
si R₁, R₂ = -H et R₄, R₆ = -H, x = 3 et y = 4, alors m : n se situe entre 65 : 35 et 45 : 55, de manière particulièrement préférée, entre 60 : 40 et 50 : 50, 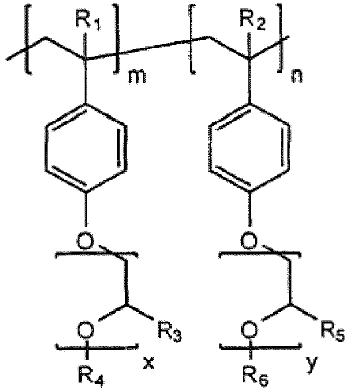
où R₁, R₂ = -H, un groupe -alkyle, de préférence, -H et -CH₃, de manière particulièrement préférée, -H,
où R₃, R₅ = -H, un groupe -alkyle, alcényle, alcinyle, aryle, de préférence, -H et -CH₃, de manière particulièrement préférée, -H, dans lequel
si R₃ ≠ -H, alors R₅ = -H,
où R₄, R₆ = -H, un groupe -alkyle, alcényle, alcinyle, aryle, de préférence -H et/ou -CH₃, de manière particulièrement préférée, -H, 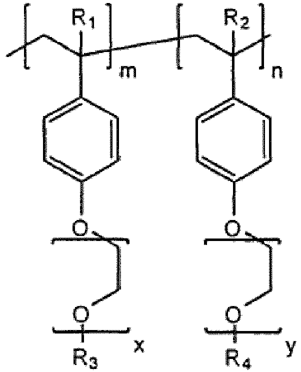
où R₁, R₂ = H ou CH₃, R₃, R₄ = H ou un groupe alkyle, x, y = 2 à 20, 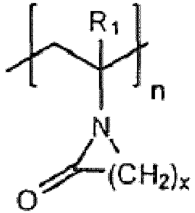
où R₁ = H ou CH₃, x = 3 à 5, des copolymères où x = 3 et x > 3, 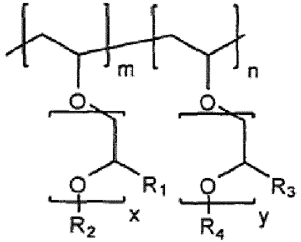
où R₁, R₃ = -H, un groupe -alkyle, alcényle, alcinyle, aryle, de préférence -H et -CH₃, de manière particulièrement préférée, -H, dans chaque cas,
si R₃, ≠ -H, alors R₅ = -H,
où R₂, R₄ = -H, un groupe -alkyle, alcényle, alcinyle, aryle, de préférence -H et/ou -CH₃, de manière particulièrement préférée, -CH₃, 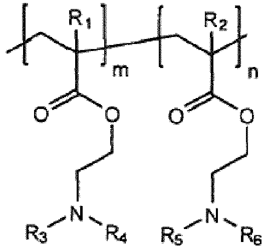 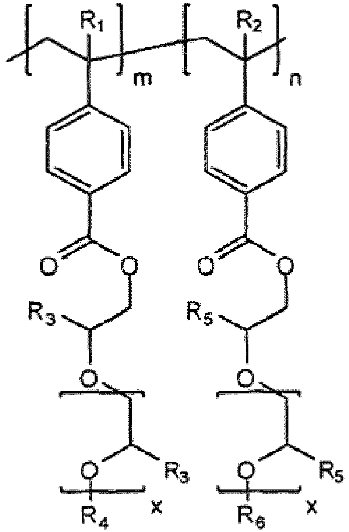
où R₁, R₂ = -H, un groupe -alkyle, de préférence - H et -CH₃, de manière particulièrement préférée, -H,
où R₃, R₅ = -H, un groupe -alkyle, alcényle, alcinyle, aryle, de préférence -H et -CH₃, de manière particulièrement préférée, -H, dans lequel,
si R₃, ≠ -H, alors R₅ = -H,
où R₄, R₆ = ≠ -H, un groupe -alkyle, alcényle, alcinyle, aryle, de préférence -H et/ou -CH₃, de manière particulièrement préférée, -H, 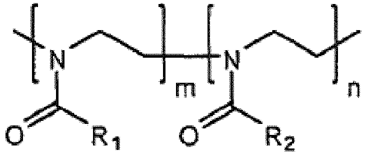
où R₁, R₂ = -H, un groupe -alkyle, alcényle, alcinyle, aryle, 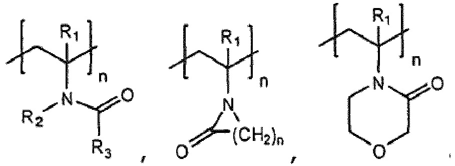
ou des copolymères des trois éléments en compositions,
où R₁ = -H, un groupe -alkyle, de préférence -H et -CH₃, de manière particulièrement préférée, -H,
où R₂, R₃ = -H, un groupe -alkyle, alcényle, alcinyle, aryle
où 2 ≤ n ≤ 10, de préférence 3 ≤ n ≤ 6, 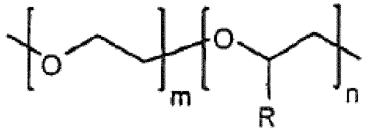
où -R = -H, un groupe -alkyle, alcényle, alcinyle, aryle, de préférence, -R = un groupe -alkyle, de manière particulièrement préférée -R = -CH₃, 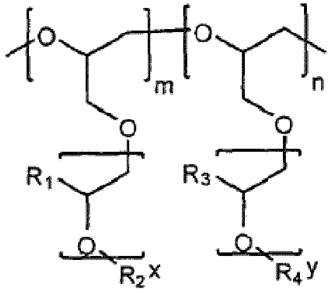
où R₁, R₃ = -H, un groupe -alkyle, alcényle, alcinyle, aryle, de préférence -H et -CH₃, dans lequel
si R₁, ≠ -H, alors R₃ = -H,
où R₂, R₄ = -H, un groupe -alkyle, alcényle, alcinyle, aryle, de préférence -H et/ou -CH₃, 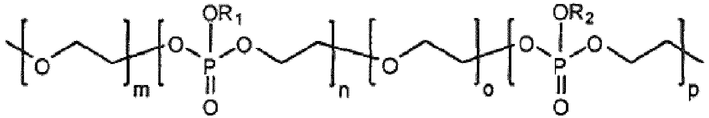
où R₁, R₂ = -H, un groupe -alkyle, alcényle, alcinyle, aryle, 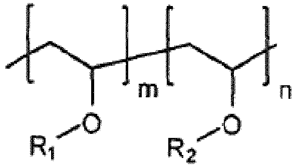
où R₁, R₂ = -H, un groupe -alkyle, alcényle, alcinyle, aryle, de préférence R₁ = -CH₃, n = 0,
et des structures analogues basées sur d'autres squelettes de polysaccharide que ceux de la cellulose, 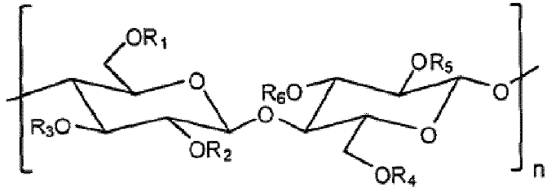
où R₁ à R₆ = -H, un groupe -alkyle, alcényle, alcinyle, aryle, -alkyloyle, au moins deux R = H,
des homopolymères ou copolymères comportant des motifs similaires à l'élastine, et des copolymères de tous les motifs mentionnés ci-dessus avec des monomères modifiés.

4. Substrat selon la revendication 3, dans lequel au moins un motif terminal de la chaîne principale de polymère contient un groupe de couplage à la surface de support (2).

5. Substrat selon l'une des revendications précédentes, dans lequel les microgels thermorépondeurs (3) sont formés d'au moins l'un des polymères : - poly-(N-isopropylacrylamide),
- -X-(-CH₂-CR₁COO-R₂-)ₙ-(-CH₂-CR₁COO-R₃)ₘ-R₄ ou des copolymères de ceux-ci, et
- -X-[(-CH₂-CR₁COO-R₂-)ₙ-(-CH₂-CR₁COO-R₃-)ₘ-R₄]₂ ou des copolymères de ceux-ci
X est un groupe de couplage à une surface de support, R₁ = H ou CH₃, R₂/R₃ = des chaînes hydrocarbonées aliphatiques avec des groupes éther et R₄H, une chaîne hydrocarbonée aliphatique ou un groupe fonctionnel.

6. Substrat selon l'une des revendications précédentes, dans lequel
- les microgels thermorépondeurs (3) sont formés d'au moins deux polymères différents et/ou présentent différents diamètres.

7. Substrat selon l'une des revendications précédentes, dans lequel
- les microgels thermorépondeurs (3) présentent un diamètre qui est d'au moins 50 nm et/ou d'au plus 10 µm.

8. Substrat selon l'une des revendications précédentes, dans lequel
- les microgels thermorépondeurs (3) présentent une structure noyau-coque.

9. Substrat selon la revendication 8, comportant au moins l'une des caractéristiques suivantes :
- la coque exclusivement des microgels thermorépondeurs (3) est thermorépondeuse,
- la cohésion des noyaux des microgels thermorépondeurs (3) est due à des interactions de valences secondaires,
- la cohésion des noyaux des microgels thermorépondeurs (3) est due à une réticulation chimique,
- des chaînes de polymère dans la coque des microgels thermorépondeurs (3) ne sont pas réticulées,
- des chaînes de polymère dans la coque des microgels thermorépondeurs (3) sont réticulées, dans lequel le nombre de points de réticulation n'est pas supérieur à 1 pour 20 motifs répétitifs non réticulés, et
- l'épaisseur de la coque des microgels thermorépondeurs (3) est d'au moins 10 nm.

10. Substrat selon l'une des revendications précédentes, dans lequel
- les microgels thermorépondeurs (3) conjointement aux particules modulatrices (5.1, 5.2) forment une monocouche, en particulier une monocouche fermée.

11. Substrat selon l'une des revendications précédentes, dans lequel
- la surface de support (2) est dotée d'un agent adhésif (4).

12. Substrat selon l'une des revendications précédentes, dans lequel
- au moins l'un des microgels thermorépondeurs (3), de l'agent adhésif et de la substance modulatrice forme sur la surface de support (2) au moins un gradient de densité (6).

13. Substrat selon l'une des revendications précédentes, dans lequel
- sur la surface de support (2), au moins une cavité de culture (9) est prévue.

14. Substrat selon l'une des revendications précédentes, dans lequel
- le corps de substrat (1) fait partie d'un dispositif de culture (30).

15. Procédé de production d'un substrat (10) selon l'une des revendications précédentes, comportant les étapes suivantes :
- mise à disposition du corps de substrat (1) avec la surface de support (2),
- production d'une dispersion des microgels thermorépondeurs (3),
- application de la dispersion sur la surface de support (2), et
- fixation des microgels thermorépondeurs (3) sur la surface de support (2),
dans lequel le procédé comprend en outre l'application d'au moins une substance modulatrice (5) sur des particules modulatrices sur la surface de support (2).

16. Procédé selon la revendication 15, comportant au moins l'une des étapes suivantes
- application d'un agent adhésif (4) sur la surface de support, et
- stérilisation de la surface de support (2).

17. Procédé de culture de cellules biologiques (20, 21, 22) sur un substrat (10) selon l'une des revendications 1 à 14, comportant les étapes suivantes :
- dépôt des cellules biologiques (20, 21, 22) sur le substrat (10), et
- ajustement des conditions de culture, de telle sorte que les cellules biologiques (20) soient soumises à une croissance, une différenciation et/ou une migration (7).

18. Procédé selon la revendication 17, comportant au moins l'une des étapes suivantes
- ajustement de l'adhérence des cellules biologiques (20, 21, 22) sur le substrat (10) par un ajustement de la température,
- ajustement d'une migration spécifique à un type de cellules (7) d'au moins un type de cellules biologiques (20, 21, 22) au moyen d'un gradient de densité d'une substance modulatrice agissant spécifiquement sur le type de cellule, et
- ajustement de la migration (7) d'au moins un type de cellules biologiques (20, 21, 22) au moyen d'un gradient de densité d'une substance modulatrice de telle sorte que les cellules biologiques (20, 21, 22) migrent dans une cavité de culture (9).
